# EUROPÄISCHE PATENTSCHRIFT

(11) **EP 1 421 052 B1**
(45) Veröffentlichungstag und Bekanntmachung des Hinweises auf die Patenterteilung: **06.06.2007**
(21) Anmeldenummer: 02764771.8
(22) Anmeldetag: 24.07.2002
(51) Int. Cl.: C07C 51/09, C07C 51/56, C07C 67/02, C07C 53/02, C07C 53/12, C07C 69/14

(54) **FLEXIBLES VERFAHREN ZUR GEMEINSAMEN HERSTELLUNG VON AMEISENSÄURE SOWIE EINER CARBONSÄURE MIT MINDESTENS ZWEI KOHLENSTOFFATOMEN UND/ODER DEREN DERIVATEN**
FLEXIBLE METHOD FOR THE COMMON PRODUCTION OF FORMIC ACID, A CARBOXYLIC ACID HAVING AT LEAST TWO CARBON ATOMS, AND/OR THE DERIVATIVES OF THE SAME
PROCEDE FLEXIBLE DE PRODUCTION SIMULTANEE D'ACIDE FORMIQUE ET D'UN ACIDE CARBOXYLIQUE AYANT AU MOINS DEUX ATOMES DE CARBONE ET/OU LEURS DERIVES

(30) Priorität: 07.08.2001 DE 10138778
(43) Veröffentlichungstag der Anmeldung: 26.05.2004
(73) Patentinhaber: BASF Aktiengesellschaft, 67056 Ludwigshafen (DE)
(72) Erfinder: ZEHNER, Peter, 67071 Ludwigshafen (DE); PASTRE, Jörg, 64625 Bensheim (DE); STÜER, Wolfram, 68165 Mannheim (DE)
(86) Internationale Anmeldenummer: PCT/EP2002/008232
(87) Internationale Veröffentlichungsnummer: WO 2003/014053

(56) Entgegenhaltungen:
- EP-A- 0 087 870
- EP-A- 0 193 799
- EP-A- 0 336 216

## Beschreibung

Die vorliegende Erfindung betrifft ein Verfahren zur gemeinsamen Herstellung von Ameisensäure sowie einer Carbonsäure mit mindestens zwei Kohlenstoffatomen und/oder deren Derivaten, wie beispielsweise Carbonsäureester, Carbonsäureanhydride oder Ketene. Insbesondere betrifft die Erfindung ein Verfahren zur Herstellung von Ameisensäure sowie Essigsäuremethylester, Essigsäureanhydrid, Essigsäure, Keten und/oder Essigsäurevinylester.

Ameisensäure ist eine wichtige und vielseitig einsetzbare Verbindung. Sie wird beispielsweise zum Ansäuren bei der Herstellung von Futtermitteln, als Konservierungsmittel, als Desinfektionsmittel, als Hilfsstoff in der Textil- und Lederindustrie sowie als Synthesebaustein in der chemischen Industrie verwendet.

Im Folgenden seien die wichtigsten Verfahren zur Herstellung von Ameisensäure genannt (siehe Ullmann's Encyclopedia of Industrial Chemistry, 6^{th} edition, 2000 electronic release, Chapter "FORMIC ACID - Production").

Das technisch bedeutendste Verfahren zur Herstellung von Ameisensäure ist die Hydrolyse von Ameisensäuremethylester (Methylformiat) und anschließender Aufkonzentrierung der erhaltenen wässrigen Ameisensäurelösung. Als bekannte Verfahren seien das Kemira-Leonard- und das BASF-Verfahren genannt. Ein großer Nachteil dieser Verfahren ist die Bildung einer wässrigen Ameisensäurelösung infolge des Hydrolyseschritts, welche eine Reihe weiterer Nachteile nach sich zieht. So ist eine aufwendige Aufkonzentrierung der Ameisensäurelösung durch Extraktivrektifikation unter Einsatz eines Schleppmittels erforderlich. Durch die Gegenwart von Wasser ist die zu handhabende wässrige beziehungsweise aufkonzentrierte Ameisensäurelösung höchst korrosionsaggressiv und erfordert den Einsatz teuerer Konstruktionsmaterialien für den betreffenden Anlagenteil. Die genannten Verfahren zeichnen sich somit nachteilig durch hohe Investitions- und Betriebskosten, durch eine technisch aufwendige und umfangreiche Gestaltung der Produktionsanlage, durch einen hohen Energieverbrauch und durch die Existenz eines nicht unerheblichen Restwassergenalts in der aufkonzentrierten Ameisensäure aus.

Bei der Oxidation von Kohlenwasserstoffen, wie beispielsweise Butane oder Naphtha, bildet sich ein breites Produktsprektrum, welches auch Ameisensäure enthält und in aufwendiger Art und Weise abgetrennt und aufkonzentriert werden kann. Auch bei diesem Verfahren ist zu dessen Nachteil eine Extraktivrektifikation der Roh-Ameisensäure unter Einsatz eines Schleppmittels erforderlich. Auf die oben genannten Nachteile infolge des Wassergehalts sei verwiesen.

Nach einem älteren Verfahren wird Ameisensäure auch durch Hydrolyse von Formamid, welches über Ammonolyse von Ameisensäuremethylester mit Ammoniak zugänglich ist, gewonnen werden. Die Hydrolyse erfolgt mit Schwefelsäure und Wasser. Nachteilig bei diesem Verfahren ist der unerwünschte Anfall von Ammoniumsulfat als Koppelprodukt und die Gegenwart von Wasser, was zu den oben genannten Nachteilen führt.

Carbonsauren, wie Essigsäure und ihre höhermolekularen Homologe, sind wichtige und vielseitig einsetzbare Verbindungen. Sie werden beispielsweise zur Herstellung von Estern, Carbonsäureanhydriden, als Zusatzstoffe im Polymerbereich oder als Zwischenprodukte bei der Herstellung von Textilchemikalien, Farbstoffen, Kunststoffen, Agrar- und Pharmachemikalien verwendet. Eine besonders hohe Bedeutung zeigen die niedermolekularen Homologe Essigsäure und Propionsäure.

Im Folgenden seien die wichtigsten Verfahren zur Herstellung von Essigsäure und ihrer höhermolekularen Homologe genannt (siehe Ullmann's Encyclopedia of Industrial Chemistry, 6^{th} edition, 2000 electronic release, Chapter "ACETIC ACID - Production" und Chapter "CARBOXYLIC ACIDS, ALIPHATIC - Production").

Das technisch bedeutendste Verfahren zur Herstellung von Essigsäure ist die Carbonylierung von Methanol in Gegenwart geeigneter Carbonylierungskatalysatoren, wie beispielsweise Cobalt-, Iridium- oder Rhodium-Carbonyl-Verbindungen. Als bekannte Verfahren seien das BASF- und das Monsanto-Verfahren genannt. Nachteilig bei diesen Verfahren ist die Gegenwart von Wasser im Reaktionsmedium, welches durch die Wassergas-Shift-Reaktion von Wasser und Kohlenmonoxid zu Kohlendioxid und Wasserstoff die Ausbeute an eingesetztem Kohlenmonoxid erniedrigt. Des weiteren ist aufgrund des Wassergehalts in der destillativen Aufarbeitung ein hoher Energieaufwand nötig. Die genannten Verfahren zeichnen sich ferner durch hohe Investitions- und Betriebkosten sowie durch eine technisch aufwendige und umfangreiche Gestaltung der Produktionsanlage aus.

Bei der Oxidation von Kohlenwasserstoffen, wie beispielsweise Ethan, Butane oder Naphtha, bildet sich ein breites Produktsprektrum, welches Essigsäure und gegebenenfalls höhere Homologe enthält und in aufwendiger Art und Weise abgetrennt und aufkonzentriert werden kann. Auf die oben genannten Nachteile infolge des Wassergehalts sei verwiesen.

Die Synthese von Carbonsäuren durch Oxidation der entsprechenden Aldehyde geht auf teures Olefin als Einsatzstoff zurück. So wird Acetaldehyd technisch durch Ethen-Oxidation nach dem Wacker-Verfahren sowie seine höheren Homologe durch Hydroformylierung von Ethen, Propen, etc. gewonnen. Diese Verfahren beruhen daher auf einer wirtschaftlich unattraktiven Rohstoffbasis.

Carbonsäureester, insbesondere Essigsäuremethylester (Methylacetat), stellen wichtige Lösungsmittel dar. Essigsäuremethylester wird beispielsweise zum Lösen von Nitrocellulose oder Acetylcellulose eingesetzt. Essigsäurevinylester findet breite Anwendung in der Herstellung von Polymeren und Copolymeren.

Die Verfahren zur Herstellung von Carbonsäureestern sind sehr vielfältig (siehe Ullmann's Encyclopedia of Industrial Chemistry, 6^{th} edition, 2000 electronic release, Chapter "ESTERS, ORGANIC - Production"). Genannt seien die Veresterung von Carbonsäuren mit Alkoholen, die Umsatzung von Carbonsäurechloriden oder Carbonsäureanhydriden mit Alkoholen, die Umesterung von Carbonsäureestern, die Umsetzung von Ketenen mit Alkoholen, die Carbonylierung von Olefinen mit Kohlenmonoxid und Alkoholen, die Kondensation von Aldehyden, die Alkoholyse von Nitrilen und die oxidative Acylierung von Olefinen.

Essigsäurealkylester werden hauptsächlich durch Veresterung von Essigsäure oder Essigsäureanhydrid mit Alkanolen gewonnen. Essigsäuremethylester entsteht des weiteren als Nebenprodukt bei der Synthese von Essigsäure (siehe Ullmann's Encyclopedia of Industrial Chemistry, 6^{th} edition, 2000 electronic release, Chapter "ACETIC ACID - Production"). Eine weitere Synthesemöglichkeit für Essigsäuremethylester ist die Carbonylierung von Dimethylether (siehe Ullmann's Encyclopedia of Industrial Chemistry, 6^{th} edition, 2000 electronic release, Chapter "ACETIC ANHYDRIDE AND MIXED FATTY ACID ANHYDRIDES - Acetic Anhydride - Production"). Nachteil des letztgenannten Verfahrens ist der Einsatz von teurem Dimethylether.

Essigsäurevinylester wird durch Addition von Essigsäure an Ethin (Acetylen), durch Addition von Essigsäureanhydrid an Acetaldehyd und anschließender Spaltung des gebildeten Ethyliden-diacetats sowie durch oxidative Acylierung von Ethen mit Essigsäure in Gegenwart von Sauerstoff gewonnen (siehe Ullmann's Encyclopedia of Industrial Chemistry, 6^{th} edition, 2000 electronic release, Chapter "VINYL ESTERS - Vinyl Acetate - Production").

GB-A 2 013 184 lehrt ein integriertes Verfahren zur Herstellung von Essigsäurevinylester aus Methanol, Kohlenmonoxid und Acetaldehyd. In einer ersten Stufe wird Essigsäure mit Methanol zum Essigsäuremethylester verestert, welcher in einer zweiten Stufe zum Essigsäureanhydrid carbonyliert wird. Das gewonnene Essigsäureanhydrid wird in einer dritten Stufe mit Acetaldehyd umgesetzt, wobei sich als Zwischenprodukt Ethyliden-diacetat bildet, was anschließend zum Essigsäurevinylester und Essigsäure zersetzt wird. Die gebildete Essigsäure wird der ersten Stufe zugeführt. Nachteilig bei diesem Verfahrens ist die Bildung stöchiometrischer Mengen von Wasser in der Veresterungsstufe und die damit vorhandene Problematik beim Umgang mit wasserhaltiger Essigsäure und deren Aufarbeitung. Zur Entfernung der gebildeten stöchiometrischen Mengen an Wasser ist in der destillativen Aufarbeitung ein hoher Energieaufwand nötig. Die verbleibende Restmenge an Wasser führt bei der anschließenden Carbonylierung infolge der Wassergas-Shift-Reaktion von Wasser und Kohlenmonoxid zu Kohlendioxid und Wasserstoff zu einer Verminderung der Ausbeute an eingesetztem Kohlenmonoxid. Die genannten Verfahren zeichnen sich ferner durch hohe Investitions- und Betriebkosten sowie durch eine technisch aufwendige und umfangreiche Gestaltung der Produktionsanlage aus.

Essigsäureanhydrid (Acetanhydrid) ist ein wichtiger Synthesebaustein in der chemischen Industrie und wird beispielsweise zur Herstellung von Acetylcellulosen, Acetylsalicylsäure, Acetanilid, Sulfonamiden oder Vitamin B6 eingesetzt.

Im Folgenden seien die wichtigsten Verfahren zur Herstellung von Essigsäureanhydrid genannt (siehe Ullmann's Encyclopedia of Industrial Chemistry, 6^{th} edition, 2000 electronic release, Chapter "ACETIC ANHYDRIDE AND MIXED FATTY ACID ANHYDRIDES - Acetic Anhydride - Production").

Ein technisch bedeutendes Verfahren zur Herstellung von Essigsäureanhydrid ist die Umsetzung von Essigsäure mit Keten, wobei das Keten in einer Vorstufe durch thermische Wasserabspaltung aus Essigsäure gewonnen wird. Nachteilig bei diesem Verfahren ist der sehr hohe Energieverbrauch durch die thermische Keten-Darstellung und der Umgang mit dem äußerst giftigen Keten.

In einem weiteren technisch bedeutenden Verfahren zur Herstellung von Essigsäureanhydrid wird in einer ersten Stufe durch Carbonylierung und Veresterung Methanol zu Essigsäuremethylester umgesetzt und dieser in einer zweiten Stufe zum Essigsäureanhydrid carbonyliert.

Ein weiteres Verfahren zur Herstellung von Essigsäureanhydrid ist die Flüssigphasen-Oxidation von Acetaldehyd. Nachteilig bei diesem Verfahren ist der Einsatz von teurem Acetaldehyd, welcher technisch durch Ethen-Oxidation nach dem Wacker-Verfahren gewonnen wird. Dieses Verfahren beruht daher auf einer wirtschaftlich unattraktiven Rohstoffbasis.

Als weiteres Verfahren zur Herstellung von Essigsäureanhydrid sei die Carbonylierung von Essigsäuremethylester in Gegenwart eines Übergangsmetall-Katalysators genannt. Essigsäuremethylester wird in der Regel als Nebenprodukt bei der Synthese von Essigsäure sowie durch Veresterung von Essigsäure mit Methanol gewonnen.
EP-A 0 087 870 lehrt ein integriertes Verfahren zur Herstellung von Essigsäureanhydrid und Essigsäure aus Methanol und Kohlenmonoxid. In einer ersten Stufe wird Essigsäure mit Methanol zum Essigsäuremethylester verestert, welcher in einer zweiten Stufe in Gegenwart von Wasser zu einem Gemisch enthaltend Essigsäureanhydrid und Essigsäure carbonyliert wird. Das gewonnene Gemisch wird destillativ aufgearbeitet, wobei die erforderliche Menge an Essigsäure der ersten Stufe zugeführt wird. Die restliche Menge an Essigsäure und an Essigsäureanhydrid wird als Produkt abgeführt. Nachteilig bei diesem Verfahren ist die Bildung stöchiometrischer Mengen von Wasser in der Veresterungsstufe und die damit vorhandene Problematik beim Umgang mit wasserhaltiger Essigsäure und deren Aufarbeitung. Auf die oben genannten Nachteile infolge des Wassergehalts sei verwiesen.

Keten ist ein wichtiges, sehr reaktives Acylierungsreagens und somit ein wichtiger Synthesebaustein. Der technisch bedeutendste Einsatz ist die Herstellung von Essigsäureanhydrid durch Umsetzung mit Essigsäure.

Technisch wird Keten hauptsächlich durch Pyrolyse von Essigsäure gewonnen. Nachteilig bei diesem Verfahren sind die erforderlichen hohen Temperaturen und der damit verbundene hohe Energieaufwand.

Weitere Verfahren sind die thermische Zersetzung von Aceton oder Essigsäureanhydrid (siehe Ullmann's Encyclopedia of Industrial Chemistry, 6^{th} edition. 2000 electronic release, Chapter "KETENES - Ketene - Production").

Es bestand daher die Aufgabe, ein Verfahren zur Herstellung von Carbonsäuren und/oder deren Derivaten zu finden, welches die oben genannten Nachteile nicht mehr besitzt, auf einer gut zugänglichen und wirtschaftlich attraktiven Rohstoffbasis basiert, eine einfache und kostengünstige Gestaltung der Anlage ermöglicht (niedrige Investitionskosten), unerwünschte Nebenprodukte infolge Koppelproduktion vermeidet und sich durch einen niedrigen Energieverbrauch und günstige Betriebskosten auszeichnet. Es bestand des weiteren die Aufgabe, ein Verfahren zu finden, welches bei Bedarf auch die Herstellung wasserfreier Carbonsäuren zugänglich macht und somit die Handhabung weniger korrosionsaggresiver Medien sowie den Einsatz kostengünstigerer Konstruktionsmaterialen ermöglicht und infolge der geringeren Korrosionsaggresivität auch eine höhere Sicherheit bietet.

Demgemäß wurde ein Verfahren zur gemeinsamen Herstellung von Ameisensäure sowie einer Carbonsäure mit mindestens zwei Kohlenstoffatomen und/oder deren Derivaten gefunden, das dadurch gekennzeichnet ist, dass man
a) einen Ameisensäureester (I) mit einer Carbonsäure mit mindestens zwei Kohlenstoffatomen (II) zu Ameisensäure (III) und dem entsprechenden Carbonsäureester (IV) umestert; und
b) mindestens einen Teil des in Schritt (a) gebildeten Carbonsäureesters (IV) zum entsprechenden Carbonsäureanhydrid (V) carbonyliert.

In Schritt (a) setzt man einen Ameisensäureester (I) mit einer Carbonsäure mit mindestens zwei Kohlenstoffatomen (II) zu Ameisensäure (III) und dem entsprechenden Carbonsäureester (IV) um.

Die einzusetzenden Ameisensäureester besitzen die allgemeine-Formel (I) 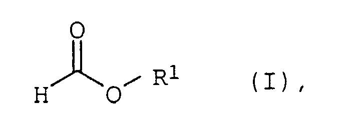 in der der Rest R¹ für einen Kohlenstoff enthaltenden organischen Rest steht. Unter einem Kohlenstoff enthaltenden organischen Rest ist bevorzugt ein unsubstituierter oder substituierter, aliphatischer, aromatischer oder araliphatischer Rest mit 1 bis 12 Kohlenstoffatomen, welcher ein oder mehrere Heteroatome, wie etwa Sauerstoff, Stickstoff oder Schwefel, wie beispielsweise -O-, -S-, -NR-, -CO- und/oder -N= in aliphatischen oder aromatischen Systemen, enthalten kann, und/oder durch eine oder mehrere funktionelle Gruppen, welche beispielsweise Sauerstoff, Stickstoff, Schwefel und/oder Halogen enthalten können, wie beispielsweise durch Fluor, Chlor, Brom, Iod und/oder eine Cyanogruppe, substituiert sein kann, zu verstehen.

Ameisensäureester sind im Allgemeinen zugänglich über eine basenkatalysierte Carbonylierung der entsprechenden Alkohole sowie über eine Veresterung der entsprechenden Alkohole mit Ameisensäure (siehe Ullmann's Encyclopedia of Industrial Chemistry, 6^{th} edition, 2000 electronic release, Chapter "FORMIC ACID - Derivatives"). Der einfachste Vertreter dieser Verbindungsklasse, Ameisensäuremethylester, wird großtechnisch durch Carbonylierung von Methanol gewonnen.

Als Carbonsäure mit mindestens zwei Kohlenstoffatomen (II) ist eine Carbonsäure zu verstehen, welche an der Carboxy-Gruppe einen Rest mit mindestens einem Kohlenstoffatom aufweist. Die einzusetzenden Carbonsäuren besitzen die allgemeine Formel (II) 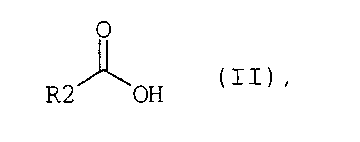 in der der Rest R² für einen Kohlenstoff enthaltenden organischen Rest, wie bei R¹ definiert, steht.

Bei der genannten Umesterungsreaktion in Schritt (a) handelt es sich um eine Gleichgewichtsreaktion, welche im Allgemeinen durch die Gegenwart eines Katalysators katalysiert wird.

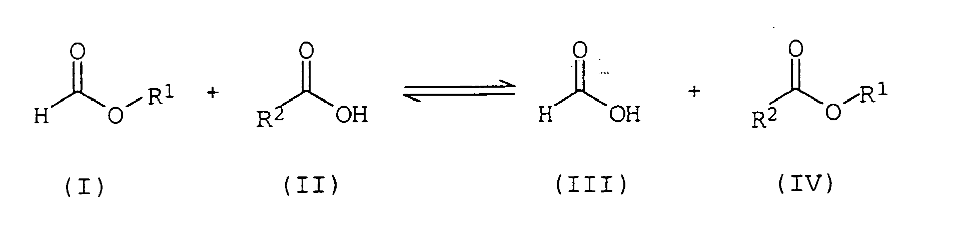

Beim erfindungsgemäßen Verfahren können in Schritt (a) die bekannten Verfahren zur Umesterung eingesetzt werden (siehe Ullmann's Encyclopedia of Industrial Chemistry, 6^{th} edition, 2000 electronic release, Chapter "ESTERS, ORGANIC - Chemical Properties" und "ESTERS, ORGANIC - Production" und die untenstehenden Zitate).

Als Katalysatoren werden im Allgemeinen geringe Mengen saurer oder basischer Substanzen eingesetzt. Bevorzugt ist der Einsatz von Säuren und sauren Festkörpern. Als Beispiele seien starke Protonensäuren, wie beispielsweise Schwefelsäure, Perchlorsäure, Benzolsulfonsäure, p-Toluolsulfonsäure, Molybdatophosphorsäure und Wolframatokieselsäure; saure Ionentauscher, wie beispielsweise perfluorierte Sulfonsäuregruppen enthaltende Ionentauscher (SU-A 1,432,048); sowie saure Oxide, wie beispielsweise Zeolithe (DE-OS 35 06 632), Aluminosilikate (US 3,328,439) oder SiO₂/TiO₂ (DE 27 10 630) genannt. Als bevorzugte Katalysatoren seien Mineralsäuren, p-Toluolsulfonsäure und Zeolithe ganannt.

Werden starke Protonensäuren als homogene Katalysatoren eingesetzt, so beträgt deren Konzentration im Reaktionsgemisch im Allgemeinen 0,01 bis 50 Gew.-%, bevorzugt 0,01 bis 2 Gew.-%.

Als Cokatalysator zu den oben genannten Katalysatoren ist der Einsatz von Wasser oder Methanol, in der Regel bis zu 20 Gew.-%, bezogen auf die Reaktionslösung, möglich. Es ist dabei aber zu beachten, dass mit Zunahme des Wassergehalts auch die Korrosionsaggressivität des Reaktionsmediums zunimmt und die Aufarbeitung der Produkte erschwert wird. Daher ist es gegebenenfalls vorteilhaft, die Umesterung ohne Zusatz von Wasser als Cokatalysator durchzuführen. Wird die Umesterung in Gegenwart von Wasser oder Methanol durchgeführt, so ist es gegebenenfalls vorteilhaft, zum Reaktionsaustrag Carbonsäureanhydrid (V) zur Bindung des Wassers zuzuführen. Dieses kann beispielsweise direkt am Reaktorausgang oder in der Kolonne (z.B. Kolonnensumpf) zugegeben werden. Durch diese Maßnahme ist auch bei einer durch Wasser oder Methanol cokatalysierten Umesterung die Darstellung von wasserfreier Ameisensäure und von wasserfreiem Carbonsäureester (IV) möglich. Auch bei einem Einsatz von methanolhaltigem Ameisensäuremethylester als Ameisensäureester (I) sind somit wasserfreie Ameisensäure und wasserfreier Carbonsäureester (IV) problemlos darstellbar. Der beim Einsatz von Ameisensäuremethylester als Ameisensäureester (I) typische Restgehalt von etwa 2 bis 4 Gew.-% Methanol erweist sich in dessen Eigenschaft als Cokatalysator als Vorteil.

Die Umesterung kann in der Flüssigphase als auch in der Gasphase durchgeführt werden. Bei der Umesterung in der Gasphase setzt man bevorzugt heterogene Katalysatoren, wie beispielsweise die genannten Ionentauscher oder sauren Oxide ein. Bei der Umesterung in der Flüssigphase verwendet man homogene oder heterogene Katalysatoren. Bevorzugt wird die Umesterung in der Flüssigphase durchgeführt.

Im Allgemeinen führt man die Umesterung bei einer Temperatur von 20 bis 300°C und bevorzugt von 50 bis 180°C durch. Der Druck beträgt in der Regel 0,1 bis 5 MPa abs.

Die Umesterung kann in Gegenwart eines zusätzlichen inerten, polaren Lösungsmittels erfolgen. Als inerte Lösungsmittel sind Lösungsmittel zu verstehen, welche unter den eingestellten Reaktionsbedingungen chemisch nicht mit den eingesetzten Verbindungen, d.h. den Edukten, den Produkten sowie den Katalysatoren, reagieren. Als geeignete Lösungsmittel seien beispielsweise Polyether ganannt. Lösungsmittel werden in der Regel bei Umesterungen eingesetzt, bei denen Edukte und/oder Produkte zugegen sind, welche bei der gewünschten Temperatur, dem gewünschten Druck und den gewünschten Mengenverhältnissen der Edukte und Produkte im lösungsmittelfreien Reaktionsgemisch nur unzureichend löslich sind. Sind die Edukte und die Produkte unter den gewählten Bedingungen auch im lösungsmittelfreien Reaktionsgemisch löslich, so führt man die Umesterung bevorzugt ohne Zusatz eines Lösungsmittels aus.

Die Edukte Ameisensäureester (I) und Carbonsäure (II) werden im Allgemeinen in jeweils stöchiometrischer Menge zugegeben.

Durch zusätzliche Zugabe eines der beiden Edukte, beispielsweise als Vorlage vor Beginn der Reaktion, kann im Reaktionsgemisch gezielt ein astöchiometrisches Verhältnis der beiden Edukte eingestellt werden. So kann beispielsweise ein Edukt, welches gute Lösungseigenschaften besitzt, die Löslichkeit des anderen Edukts oder der Produkte verbessern. Ebenso ist es auch möglich, einen entsprechenden Überschuß eines der beiden Produkte im Reaktionsgemisch aufrecht zu erhalten.

Die Umesterung kann diskontinuierlich oder kontinuierlich erfolgen. Bevorzugt ist ein kontinuierliches Verfahren.

Für die Umesterung können beim erfindungsgemäßen Verfahren prinzipiell alle, für Umesterungsreaktionen bekannten Reaktionsapparate eingesetzt werden. Als geeignete Reaktionsapparate für die Umsetzung in der Flüssigphase seien beispielsweise Rührkesselreaktoren, Destillationskolonnen, Reaktivkolonnen und Membranreaktoren genannt. Um einen hohen Umsatz zu erreichen, ist es vorteilhaft, mindestens eines der beiden Produkte, bevorzugt alle beide, stetig aus dem Reaktionsgemisch zu entfernen. Beim Einsatz eines Rührkesselreaktors wird dies beispielsweise durch eine kontinuierliche Entnahme des Reaktionsgemisches, nachfolgender Trennung der beiden Produkte und Rückführung der beiden nicht-umgesetzten Edukte sowie gegebenenfalls des Katalysators erreicht.

Beim Einsatz einer Destillationskolonne erfolgt die Umesterungsreaktion im Sumpf, wobei die leichter siedenden Komponenten destillativ getrennt und je nachdem, ob es sich um Edukt oder Produkt handelt, wieder rückgeführt oder abgeführt werden können. Beim Einsatz einer Reaktivkolonne befindet sich der bevorzugt heterogene Katalysator im Trennbereich der Kolonne. Die leichter siedenden Komponenten werden hier ähnlich der beschriebenen Destillationskolonne destillativ getrennt und rückgeführt beziehungsweise abgeführt.

Als geeignete Reaktionsapparate für die Umsetzung in der Gasphase seien beispielsweise Strömungsrohre oder Schachtreaktoren genannt.

Die Trennung des Reaktionsgemisches kann auf verschiedene Weise erfolgen. Sie wird in der Regel durch die Eigenschaften der zu trennenden Edukte und Produkte bestimmt. Als Beispiele möglicher Trennverfahren seien die Destillation, die Kristallisation und die Extraktion genannt. Es sei darauf hingewiesen, dass auch Kombinationen verschiedener Trennverfahren, auch bei Voranschaltung einer Destillations- oder Reaktivkolonne zur Umesterung, möglich sind. Bevorzugt ist im Allgemeinen die destillative Trennung, welche gegebenenfalls auch als Destillation bei Unterdruck oder im Vakuum durchgeführt werden kann. Ist eine destillative Trennung nicht oder nur unter großem Aufwand möglich, beispielsweise bei höhersiedenden oder leicht zersetzlichen Komponenten, gewinnen die genannten alternativen Verfahren an Bedeutung. Bei Kenntnis der vorliegenden Edukte, Produkte und gegebenenfalls des Katalysators ist es für den Fachmann ohne Weiteres möglich, ein geeignetes Aufarbeitungskonzept zu entwickeln.

Ameisensäure (III) wird aufgrund ihrer guten Destillationseigenschaften in der Regel destillativ entfernt.

Bei der bevorzugten destillativen Trennung des erhaltenen Reaktionsgemisches werden in der Regel drei Destillationskolonnen beziehungsweise deren Äquivalente (z.B. eine Trennwandkolonne und eine Destillationskolonne) eingesetzt, um eine Trennung in vier Ströme zu erhalten. Der Ameisensäureester (I) enthaltende Strom wird im Allgemeinen zur Umesterung zurückgeführt, der Carbonsäureester (IV) enthaltende Strom wird teilweise oder ganz dem Carbonylierungsschritt (b) zugeführt, die Ameisensäure (III) wird aus dem System als Produkt abgeführt und der verbleibende, die Carbonsäure (II) enthaltende Strom wird im Allgemeinen ebenfalls zur Umesterung zurückgeführt.

Da bei der nachfolgenden Carbonylierung des Carbonsäureesters (IV) zum Carbonsäureanhydrid (V) in Gegenwart des Carbonylierungskatalysators gegebenenfalls noch vorhandener Ameisensäureester (I) zur entsprechenden Carbonsäure R¹-COOH isomerisiert wird, ist es in einer Variante mit vereinfachter destillativer Aufarbeitung unter Einsparung einer Destillationskolonne gegebenenfalls möglich, neben einem Ameisensäureester (I) enthaltenden Strom, einem Ameisensäure (III) enthaltenden Strom und einen Carbonsäure (II) enthaltenden Strom, als weiteren Strom einem Ameisensäureester (I) und Carbonsäureester (IV) enthaltenden Strom zu gewinnen und diesen dem Carbonylierungsschritt (b) zuzuführen. Dieser letztgenannte Strom kann beispielsweise als Seitenabzug der ersten Destillationskolonne gewonnen werden.

Beim erfindungsgemäßen Verfahren kann die gesamte Menge des erhaltenen Carbonsäureesters (IV) oder auch nur ein Teil davon dem Carbonylierungsschritt (b) zugeführt werden. Bei der letztgenannten Variante kann ein Teil des gebildeten Carbonsäureesters (IV) als Endprodukt erhalten werden. Der verbleibende Teil des Carbonsäureesters (IV) wird dem Carbonylierungsschritt (b) zugeführt.

In Schritt (b) carbonyliert man mindestens einen Teil, bevorzugt mindestens 5%, besonders bevorzugt mindestens 10% und ganz besonders bevorzugt mindestens 50% des in Schritt (a) gebildeten Carbonsäureesters (IV) in Gegenwart eines Katalysators zum entsprechenden Carbonsäureanhydrid (V). Es ist auch möglich, die gesamte, in Schritt (a) gebildete Menge an Carbonsäureester (IV) im Schritt (b) zu carbonylieren.

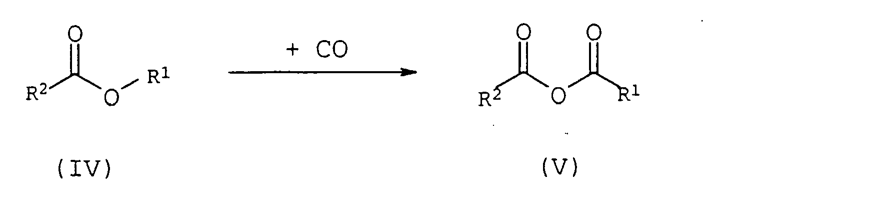

Beim erfindungsgemäßen Verfahren können in Schritt (b) die bekannten Verfahren zur Carbonylierung von Carbonsäureestern eingesetzt werden (siehe Ullmann's Encyclopedia of Industrial Chemistry, 6^{th} edition, 2000 electronic release, Chapter "ACETIC ANHYDRIDE AND MIXED FATTY ACID ANHYDRIDES - Acetic Anhydride - Production" und die untenstehenden Zitate).

Als Katalysatoren können im Allgemeinen Metalle der 8. bis 10. Gruppe des Periodensystems sowie deren Verbindungen in Gegenwart von Halogeniden sowie organischen Halogenverbindungen eingesetzt.

Als bevorzugte Katalysatormetalle seien Rhodium, Iridium, Palladium, Nickel und Cobalt, insbesondere Rhodium, genannt (EP-A 0 677 505). Als Halogenide beziehungsweise organische Halogenverbindungen werden in der Regel Iodverbindungen eingesetzt. Bevorzugt ist die Zugabe von Alkali- und Erdalkaliiodiden (US 5,003,104, US 4,559,183), Iodwasserstoffsäure, Iod, Iodalkane, insbesondere Iodmethan (Methyliodid) (GB-A 2,333,773, DE-OS 24 41 502) oder substituiertes Azoliumiodid (EP-A 0 479 463). Die Katalysatormetalle sind in der Regel durch Liganden stabilisiert. Als geeignete Liganden werden bevorzugt Stickstoff- und Phosphorverbindungen, wie beispielsweise N-haltige heterocyclische Verbindungen (DE-OS 28 36 084), Amine, Amide (DE-OS 28 44 371) oder Phosphine (US 5,003,104, EP-A 0 336 216) eingesetzt. Die Katalysatorsysteme können ferner noch Promotormetalle, wie beispielsweise Chrom im System Nickel/Chrom (US 4,002,678), Ruthenium im System Iridium/Ruthenium (GB-A 2,333,773) oder Cobalt im System Ruthenium/Cobalt (US 4,519,956) enthalten. Als bevorzugte Katalysatorsysteme seien Systeme welche Rhodium und/oder Iridium, Methyliodid, Stickstoff- und/oder Phosphor-enthaltende Liganden sowie gegebenenfalls Promotoren, wie beispielsweise Lithium oder Chrom, enthalten, genannt. Besonders bevorzugt ist der Einsatz eines Katalysators auf Basis Rhodiumtriiodid, Lithiumiodid und Iodmethan, wie beispielsweise in US 4,374,070 beschrieben.

Der Katalysator kann ungeträgert als sogenannter Homogenkatalysator oder geträgert als sogenannter Heterogenkatalysator eingesetzt werden. Als geeignete Trägermaterialien seien beispielhaft anorganische Oxide, wie etwa Siliziumdioxid oder Aluminiumoxid (EP-A 0 336 216), oder Polymere, wie etwa Ionentauscher (J6 2135 445) oder Harze (JP 09 124 544) genannt.

Die Carbonylierung kann in Gegenwart von Wasserstoff (US 5,003,104, GB-A 2 333 773, US 4,333,885, WO 82/01704) oder in Abwesenheit von Wasserstoff (A.C. Marr et al., Inorg. Chem. Comm. 3, 2000, Seite 617 bis 619) durchgeführt werden. Im Allgemeinen ist es vorteilhaft, die Carbonylierung in Gegenwart von Wasserstoff durchzuführen, wobei man in der Regel Wasserstoffkonzentrationen vom ppm-Bereich bis hin zu 15 Vol.-% und bevorzugt von 1 bis 10 Vol.-%, bezogen auf den zugeführten gasförmigen Eduktstrom, wählt.

Die Carbonylierung kann sowohl in der Gasphase (EP-A 0 336 216) als auch in der Flüssigphase durchgeführt werden. Bei einer Durchführung in der Gasphase setzt man im Allgemeinen geträgerte Katalysatoren ein. Bevorzugt ist beim erfindungsgemäßen Verfahren die Carbonylierung in der Flüssigphase.

Die Carbonylierung in der Gasphase führt man im Allgemeinen bei einer Temperatur von 130 bis 400°C, bevorzugt von 150 bis 280°C und einem Druck von 0,1 bis 15 MPa abs, bevorzugt 0,5 bis 3 MPa abs durch. Die Carbonylierung in der Flüssigphase führt man im Allgemeinen bei einer Temperatur von 100 bis 300°C, bevorzugt von 170 bis 200°C und einem Druck von 0,1 bis 15 MPa abs, bevorzugt 1 bis 8 MPa abs durch.

Bei der bevorzugten Carbonylierung in der Flüssigphase und dem Einsatz eines Homogenkatalysators setzt man in der Regel eine Katalysatorkonzentration im Bereich von 0,01 bis 1 Gew.-% bezogen auf die Reaktionslösung ein.

Die Carbonylierung kann in Gegenwart eines zusätzlichen inerten Lösungsmittels erfolgen. Als inerte Lösungsmittel sind Lösungsmittel zu verstehen, welche unter den eingestellten Reaktionsbedingungen chemisch nicht mit den eingesetzten Verbindungen, d.h. den Edukten, den Produkten sowie den Katalysatoren, reagieren. Geeignete inerte Lösungsmittel sind beispielsweise aromatische und aliphatische Kohlenwasserstoffe sowie Carbonsäuren oder deren Ester. Bevorzugt werden Lösungsmittel bei Carbonylierungen eingesetzt, bei denen das Edukt und/oder das Produkt bei der gewünschten Temperatur und/oder dem gewünschten Druck im lösungsmittelfreien Reaktionsgemisch nur unzureichend löslich sind. Sind die Edukte und die Produkte unter den gewählten Bedingungen auch im lösungsmittelfreien Reaktionsgemisch löslich, so führt man die Carbonylierung bevorzugt ohne Zusatz eines Lösungsmittels aus.

Die Carbonylierung kann diskontinuierlich oder kontinuierlich erfolgen. Bevorzugt ist ein kontinuierliches Verfahren.

Für die Carbonylierung können beim erfindungsgemäßen Verfahren prinzipiell alle, für Carbonylierungsreaktionen bekannten Reaktionsapparate eingesetzt werden. Die Carbonylierung in der Gasphase führt man im Allgemeinen in einem Strömungsrohr oder Schachtreaktor durch. Als geeignete Reaktionsapparate für die bevorzugte Carbonylierung in der Flüssigphase seien beispielsweise Rührkesselreaktoren, Strahlschlaufenreaktoren und Blasensäulen genannt. Im Folgenden ist deren Einsatz in einem kontinuierlichen Verfahren kurz beschrieben.

Beim Einsatz der genannten Reaktionsapparate führt man in der Regel die gewünschten Mengen an Carbonsäureester (IV) und Kohlenmonoxid kontinuierlich zur Reaktionslösung, welche insbesondere das Carbonsäureanhydrid (V), den Carbonylierungskatalysator und gegebenenfalls ein zusätzliches Lösungsmittel enthält, unter intensiver Durchmischung zu. Die gebildete Carbonylierungswärme kann beispielsweise durch innenliegende Wärmetauscher, durch Kühlung der Wandung des Reaktionsapparats und/oder durch kontinuierliche Entnahme der heißen Reaktionslösung, außenliegende Kühlung und Rückführung entzogen werden. Beim Einsatz eines Strahlschlaufenreaktors oder einer Blasensäule ist zur Sicherstellung der Durchmischung ein externer Kreislauf erforderlich. Die Produktabfuhr erfolgt durch kontinuierliche Entnahme und anschließende Abtrennung des Carbonylierungskatalysators in einer geeigneten Abtrennvorrichtung. Als geeignete Abtrennvorrichtung sei beispielsweise ein sogenannter Flash-Verdampfer genannt, bei dem das Carbonsäureanhydrid (V) durch Druckentspannung verdampft. Die verbleibende Lösung, welche den Carbonylierungskatalysator enthält, wird dem Reaktionsapparat wieder zugeführt. Durch geeignete Temperatur- und Druckführung ist es gegebenenfalls auch möglich, das gebildete Carbonsäureanhydrid durch Verdampfung aus der Reaktionslösung kontinuierlich abzuziehen (DE-OS 30 24 353). Das verdampfte Carbonsäureanhydrid (V) kann je nach Erfordernis einer Aufarbeitungsstufe oder einer Folgestufe zur weiteren Umsetzung zugeführt werden. Bei höhersiedenden Carbonsäureanhydriden (V), bei denen die beschriebene Flashverdampfung aufgrund deren geringer Flüchtigkeit nicht möglich ist, ist der Reaktionsaustrag durch andere Maßnahmen, beispielsweise destillativ unter Unterdruck, durch Kristallisation oder Extraktion aufzuarbeiten.

Die beim erfindungsgemäßen Verfahren zu wählenden Verfahrensparameter und Maßnahmen sind unter anderem von der Natur des eingesetzten Carbonsäureesters (IV), des gebildeten Carbonsäureanhydrids (V) und dem ausgewählten Katalysatorsystem abhängig und mit dem üblichen Fachkönnen zu ermitteln.

Abhängig von den gewählten Edukten Ameisensäureester (I) und Carbonsäure (II) wird durch die Carbonylierung in Schritt (b) ein symmetrisches oder asymmetrisches Carbonsäureanhydrid gebildet, d.h. die Reste R¹ und R² können identisch oder verschieden sein.

Des weiteren ist es möglich, zu dem zu carbonylierenden Carbonsäureester (IV) einen Alkohol R¹-OH oder R²-OH zuzuführen. Der Alkohol wird dabei zur entsprechenden Carbonsäure R¹-COOH (VIIb) beziehungsweise R²-COOH (II) umgesetzt. Durch eine derartige Zufuhr ist es möglich, das Verhältnis zwischen den Carbonylierungsprodukten R²-COOH (II), Carbonsäureanhydrid (V) und R¹-COOH (VIIb) zur Ameisensäure (I) zu erhöhen. So führt beispielsweise die zusätzliche Zufuhr von Methanol bei der Carbonylierung von Essigsäuremethylester (IV) zur Bildung von Essigsäure neben Essigsäureanhydrid aus der Carbonylierung des Essigsäuremethylesters (IV). Ferner ist es auch möglich, dem zu carbonylierenden Carbonsäureester (IV) als weitere Komponente zusätzlich Wasser, Carbonsäureester (IV), Ameisensäureester (I) oder Ether der allgemeinen Formeln R¹-O-R¹, R¹-O-R² oder R²-O-R² zuzuführen.

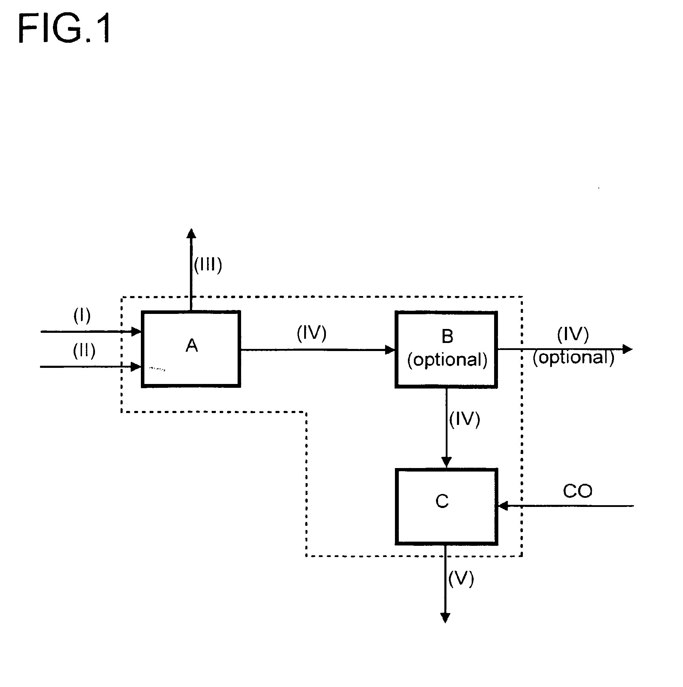

Abbildung 1 zeigt ein Blockdiagramm des erfindungsgemäßen Verfahrens. Ameisensäureester (I) und Carbonsäure (II) werden in Block "A" (Umesterung/Trennung) unter Bildung von Ameisensäure (III) und Carbonsäureester (IV) umgesetzt. Die abgetrennt Ameisensäure (III) wird als Endprodukt abgeführt. Der abgetrennte Carbonsäureester (IV) wird über einen optional vorhandenen Block "B" (Ausschleusung Carbonsäureester), bei dem gegebenenfalls ein Teil des gebildeten Carbonsäureesters (IV) als Endprodukt ausgeschleust werden kann, dem Block "C" (Carbonylierung) zugeführt. Unter Zufuhr von Kohlenmonoxid wird Carbonsäureanhydrid (V) gebildet, welches als Endprodukt ausgeschleust oder als Zwischenprodukt einer optionalen Folgestufe zugeführt werden kann.

In einer bevorzugten Ausführungsform des erfindungsgemäßen Verfahrens überführt man in Schritt (c) mindestens einen Teil, bevorzugt mindestens 5%, besonders bevorzugt mindestens 10% und ganz besonders bevorzugt mindestens 50% des in Schritt (b) gebildeten Carbonsäureanhydrids (V) in die Carbonsäure (II). Geeignet sind hierzu prinzipiell alle Verfahren, welche zur Bildung der Carbonsäure (II) führen. Die genannte Überführung erfolgt im Allgemeinen (i) durch thermische Zersetzung, (ii) durch Hydrolyse, (iii) durch den Einsatz als Acylierungsreagens und/oder (iv) durch Hydrierung.

Im Folgenden seien die genannten Reaktionswege näher erläutert.

### (i) thermische Zersetzung

Die thermischen Zersetzung des Carbonsäureanhydrids (V) zur entsprechenden Carbonsäure und einem Keten ist prinzipiell bei einem Carbonsäureanhydrid (V) möglich, welches mindestens ein Wasserstoffatom in α-Stellung zur Carboxycarbonyl-Gruppe aufweist. Im Folgenden sei die allgemeine Reaktionsgleichung zur Zersetzung des Carbonsäureanhydrids (V) in die Carbonsäure (II) und einem Keten dargestellt 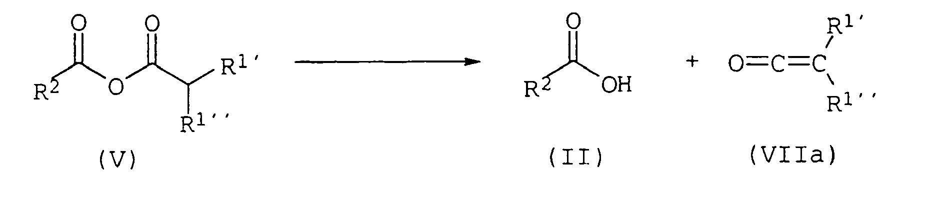 wobei der Rest (R1')(R1")CH unter die allgemeine Definition des Restes R¹ fällt.

Keten ist als "monomeres Carbonsäureanhydrid" zu verstehen und fällt somit unter den Begriff Carbonsäurederivat.

Bevorzugt ist ein Verfahren zur Herstellung eines Ketens, bei dem man durch entsprechende Wahl des Ameisensäureesters (I) und der Carbonsäure (II) in Schritt (a) als Carbonsäureanhydrid (V) bei Schritt (c) ein symmetrisches Carbonsäureanhydrid, welches mindestens ein Wasserstoffatom in α-Stellung zur Carboxycarbonyl-Gruppe und/oder eine Acetyl-Gruppe enthaltendes Carbonsäureanhydrid, insbesondere Essigsäureanhydrid, einsetzt.

Die thermische Zersetzung kann im Allgemeinen bei einer Temperatur im Bereich 300 bis 1000°C und bevorzugt 350 bis 800°C und einem Druck von 0,01 bis 1,0 MPa abs in der Gasphase in Gegenwart oder Abwesenheit eines Katalysator durchgeführt werden (siehe US 2,045,739, WO 93/04026 und G.J. Fisher et al., J. Org. Chem. 18, 1954, Seiten 1055 bis 57). Das gebildete Reaktionsgas, welches Keten, Carbonsäure (II), nicht-umgesetztes Carbonsäureanhydrid (V) sowie Nebenprodukte wie beispielsweise Kohlendioxid oder Methan enthält, sollte zur Vermeidung der Rekombination zum Carbonsäureanhydrid (V) möglichst schnell abgekühlt werden.

Die konkret zu wählenden Verfahrensparameter und Maßnahmen sind unter anderem von der Natur des eingesetzten Carbonsäureanhydrids (V), der gebildeten Reaktionsprodukte und dem gegebenenfalls ausgewählten Katalysator abhängig und mit dem üblichen Fachkönnen zu ermitteln.

Der gasförmige Reaktionsaustrag, welcher das Keten (VIIa), die Carbonsäure (II) sowie gegebenenfalls nicht-umgesetztes Carbonsäureanhydrid (V) und Nebenprodukte enthält, wird beispielsweise ein- oder mehrstufig abgekühlt, so dass die gebildete Carbonsäure (II) auskondensiert. Das Keten (VIIa) liegt unter diesen Bedingungen in der Regel gasförmig vor und wird gasförmig ausgeschleust.

In einer anderen Variante wird der gasförmige Reaktionsaustrag beispielsweise einem sogenannten Waschturm zugeführt, wobei der Reaktionsaustrag gegebenenfalls durch eine vorgeschaltete Kühlzone vorgekühlt wird. Das im Waschturm eingesetzte Lösungsmittel entspricht bevorzugt der gebildeten Carbonsäure (II). Im Allgemeinen wird der Waschturm derart betrieben, dass die zugeführte Carbonsäure (II) ausgewaschen und das Keten gasförmig aus dem Waschturm abgeführt wird. Eine der gebildeten Carbonsäure (II) entsprechende Menge wird in der Regel kontinuierlich dem Waschturm entnommen .

Bei der Bildung eines höhermolekularen Ketens (VIIa) und/oder einer höhermolekularen Carbonsäure (II) ist gegebenenfalls auch eine Kondensation der Reaktionsgase oder eine Wäsche mit einem weiteren Lösungsmittel möglich.

### (ii)Hydrolyse

Bei der Hydrolyse des Carbonsäureanhydrids (V) bildet sich die entsprechende Carbonsäure (VIIb) und die Carbonsäure (II).

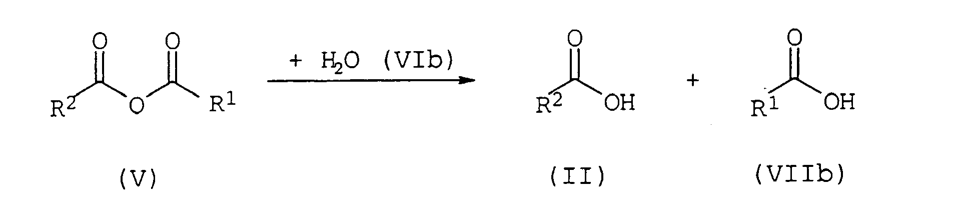

Beim erfindungsgemäßen Verfahren können bei der Hydrolyse in Schritt (c) prinzipiell alle geeigneten Verfahren zur Hydrolyse von Carbonsäureanhydriden eingesetzt werden.

Die Hydrolyse kann in Gegenwart oder Abwesenheit eines Katalysators durchgeführt werden. Werden durch die Hydrolyse des Carbonsäureanhydrids (V) starke Carbonsäuren gebildet, wie beispielsweise Essigsäure, so kann aufgrund deren katalytischer Wirkung gegebenenfalls auf die Gegenwart eines weiteren Katalysators verzichtet werden. Werden hingegen schwache Carbonsäuren gebildet, wie beispielsweise Butansäure (Buttersäure), so ist in der Regel der Einsatz eines Katalysators zu empfehlen.

Als Katalysatoren werden im Allgemeinen geringe Mengen an Säuren oder sauren Festkörpern eingesetzt. Prinzipiell sind die für die Umesterung in Schritt (a) genannten Säuren und sauren Festkörpern auch für den Einsatz in der Hydrolyse geeignet, worauf hiermit explizit verwiesen sei. Als bevorzugte Katalysatoren seien Mineralsäuren, p-Toluolsulfonsäure und Zeolithe ganannt.

Werden starke Protonensäuren als homogene Katalysatoren eingesetzt, so beträgt deren Konzentration im Reaktionsgemisch im Allgemeinen 0,001 bis 50 Gew.-%, bevorzugt 0,01 bis 10 Gew.-% und besonders bevorzugt 0,01 bis 2 Gew.-%.

Die Hydrolyse kann in der Flüssigphase als auch in der Gasphase durchgeführt werden. Bei der Hydrolyse in der Gasphase setzt man bevorzugt heterogene Katalysatoren, wie beispielsweise die genannten Molekularsiebe, Ionentauscher oder saure Oxide ein. Bei der Hydrolyse in der Flüssigphase verwendet man homogene oder heterogene Katalysatoren. Bevorzugt wird die Hydrolyse in der Flüssigphase durchgeführt.

Im Allgemeinen führt man die Hydrolyse bei einer Temperatur von 20 bis 300°C und bevorzugt von 50 bis 180°C durch. Der Druck beträgt in der Regel 0,1 bis 10 MPa abs und bevorzugt 0,1 bis 5 MPa abs.

Die Hydrolyse kann auch in Gegenwart eines zusätzlichen inerten Lösungsmittels durchgeführt werden. Als inerte Lösungsmittel sind Lösungsmittel zu verstehen, welche unter den eingestellten Reaktionsbedingungen chemisch nicht mit den eingesetzten Verbindungen, d.h. den Edukten, den Produkten sowie den Katalysatoren, reagieren. Als inerte Lösungsmittel geeignet sind beispielsweise jene Lösungsmittel, welche bei der Umesterung (Schritt (a)) beschrieben sind.

Die Edukte Carbonsäureanhydrid (V) und Wasser (VIb) werden im Allgemeinen in jeweils stöchiometrischer Menge zugegeben, so dass das Carbonsäureanhydrid (V) vollständig unter Bildung wasserfreier Carbonsäuren umgesetzt werden kann.

Die Hydrolyse kann diskontinuierlich oder kontinuierlich erfolgen. Bevorzugt ist ein kontinuierliches Verfahren.

Für die Hydrolyse können beim erfindungsgemäßen Verfahren prinzipiell alle Reaktionsapparate eingesetzt werden, welche eine intensive Durchmischung der Reaktionslösung beziehungsweise des Reaktionsgases ermöglichen, unter den sauren Bedingungen korrosionsstabil sind und die Abfuhr der gebildeten Reaktionswärme ermöglichen. Die Hydrolyse in der Gasphase führt man im Allgemeinen in einem Strömungsrohr oder Schachtreaktor durch. Als geeignete Reaktionsapparate für die Hydrolyse in der Flüssiphase seien beispielsweise Rührkesselreaktoren, Strömungsrohre mit Mischern, Destillationskolonnen und Reaktivkolonnen genannt. Im Allgemeinen führt man das Carbonsäureanhydrid (V) und die gewünschte Menge Wasser dem Reaktionsapparat kontinuierlich unter intensiver Durchmischung zu. Rührkesselreaktoren und Strömungsrohre sind in der Regel mit entsprechenden Kühlvorrichtungen ausgestattet. Beim Einsatz einer Destillations- oder Reaktivkolonne kann die gebildete Reaktionswärme vorteilhafterweise direkt zur destillativen Trennung genutzt werden. Vorteilhafterweise entnimmt man beim Einsatz eines asymmetrischen Carbonsäureanhydrids (V) einen Carbonsäure (II) haltigen und einen Carbonsäure (VIIb) haltigen Strom. Beim Einsatz eines symmetrischen Carbonsäureanhydrids (V) wird in der Regel nur ein Produktstrom entnommen, da die gebildeten Carbonsäure (II) und (VIIb) identisch sind.

Setzt man beim Einsatz eines Rührkesselreaktors oder Strömungsrohrs ein asymmetrisches Carbonsäureanhydrid (V) ein, so trennt man den kontinuierlich entnommenen Reaktoraustrag in die Carbonsäure (II) und (VIIb). Der gegebenenfalls vorhandene homogene Katalysator kann in der Regel wieder rückgeführt werden. Als Beispiele möglicher Trennungverfahren seien die Destillation, die Kristallisation und die Extraktion genannt. Es sei darauf hingewiesen, dass auch Kombinationen verschiedener Trennverfahren, auch bei Voranschaltung einer Destillations- oder Reaktivkolonne zur Hydrolyse, möglich sind. Bevorzugt ist im Allgemeinen die destillative Trennung, welche gegebenenfalls auch als Destillation bei Unterdruck oder im Vakuum durchgeführt werden kann. Ist eine destillative Trennung nicht oder nur unter großem Aufwand möglich, beispielsweise bei höhersiedenden oder leicht zersetzlichen Komponenten, gewinnen die genannten alternativen Verfahren an Bedeutung. Bei Kenntnis der Eigenschaften der gebildeten Carbonsäuren und gegebenenfalls des Katalysators ist es für den Fachmann ohne Weiteres möglich, ein geeignetes Aufarbeitungskonzept zu entwickeln.

In einer bevorzugten Variante des erfindungsgemäßen Verfahrens, bei der die Carbonsäure (II) und die Carbonsäure (VIIb) durch Hydrolyse gewonnen werden, führt man die Carbonylierung von Schritt (b) und die Hydrolyse von Schritt (c) zusammen in einem Reaktionsapparat durch. Als Reaktionsapparate eignen sich prinzipiell alle bei der Carbonylierung unter Schritt (b) beschriebenen Apparate. Bei der genannten Variante führt man dem Reaktionsapparat kontinuierlich den Carbonsäureester (IV), Kohlenmonoxid und Wasser, bevorzugt in stöchiometrischem Verhältnis, zu. Als Katalysator enthält das Reaktiongemsich einen Carbonylierungskatalysator wie bereits oben beschrieben. Des weiteren ist es möglich und bei schwächeren Säueren in der Regel erforderlich, dass das Reaktionsgemisch katalytische Mengen eines Hydrolysekatalysators wie oben beschrieben enthält. Im übrigen wird die Umsetzung wie bei der Carbonylierung beschrieben durchgeführt. Auch die Entnahme des Reaktionsgemisches und dessen Aufarbeitung erfolgt im Wesentlichen wie bei der Carbonylierung beschrieben, d.h. bevorzugt über eine Entspannungsstufe und einen sogenannten Flashverdampfer, bei dem die Produkte abgezogen werden und die verbleibende katalysatorhaltige Lösung rückgeführt wird. Falls die gebildeten Carbonsäuren (II) und (VIIb) nicht identisch sind, wird der erhaltene Produktstrom in der Regel nach den üblichen Verfahren aufgetrennt.

Es ist natürlich auch möglich, die Carbonylierung von Schritt (b) mit partieller Hydrolyse von Schritt (c) zusammen in einem Reaktionsapparat durchzuführen. Die hierbei zuzuführende Wassermenge ergibt sich dann aus dem gewünschten Anteil an der Carbonsäure (VIIb). So sind als Reaktionsprodukt auch beliebige Mischungen von Carbonsäure (VIIb) und Carbonsäureanhydrid (V) direkt zugänglich.

### (iii) Einsatz als Acylierungsreagens

Beim Einsatz als Acylierungsreagens wird formal eine Acylgruppe auf einen geeigneten Wasserstoff-aktiven Reaktionspartner übertragen, wobei die verbleibende Acyloxygruppe über eine gegebenenfalls isolierbare Zwischenstufe zur Carbonsäure umgesetzt wird. Als Wasserstoff-aktive Reaktionspartner sind Reaktionspartner zu verstehen, welche in der Lage sind, formal ein Wasserstoff-Radikal auf die Acyloxygruppe zu übertragen.

Ohne limitierend zu wirken seien die untenstehenden Reaktionen (aa) bis (ee), bei denen das Carbonsäureanhydrid (V) als Acylierungsreagens unter Bildung der Carbonsäure (II) eingesetzt werden kann, genannt. Es sei darauf hingewiesen, dass bei Einsatz eines asymmetrischen Carbonsäureanhydrids (V) in der Regel eine Acylierung mit der R¹-CO- und der R²-CO-Gruppe unter Bildung eines entsprechenden Carbonsäuregemisches enthaltend R²-COOH und R¹-COOH erfolgt. Der Einfachheit halber sind in den untenstehenden Reaktionsschemata beide Reaktionswege zusammengefaßt. Die allgemeinen Formeln in den Reaktionsschemata, welche die Reste "R1/2" enthalten, stehen für eine entsprechende Mischung von Verbindungen enthaltend die Reste R¹ sowie R².

Bevorzugt führt man die untenstehenden Reaktionen (aa) bis (ee) mit einem symmetrischen Carbonsäureanhydrid (V), da in diesem Fall beide Reste R¹ und R² identisch sind und somit nur eine Spezies an Carbonsäure, nämlich die Carbonsäure (II), und nur eine Spezies an Carbonsäurederivat gebildet werden.

### (aa) Die Umsetzung mit einem Alkohol zu einem Carbonsäureester (VIIc)

Die allgemeine Reaktionsgleichung hierzu lautet:

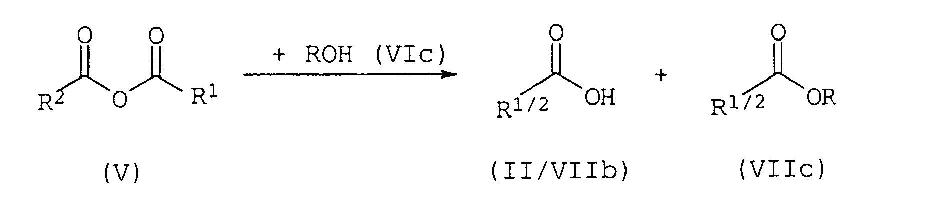

Der Rest R steht im Allgemeinen für einen Kohlenstoff enthaltenden organischen Rest. Bevorzugt steht der Rest R für
- einen unsubstituierten oder substituierten, unverzweigten oder verzweigten, acyclischen oder cyclischen C₁- bis C₁₂-Alkylrest, wie beispielsweise Methyl, Ethyl, Propyl, 1-Methylethyl, Butyl, 1-Methylpropyl, 2-Methylpropyl, 1,1-Dimethylethyl, Pentyl, Hexyl, Heptyl, 1-Ethylpentyl, Octyl, 2,4,4-Trimethylpentyl, Nonyl, 1,1-Dimethylheptyl, Decyl, Undecyl, Dodecyl, Phenylmethyl, 2-Phenylethyl, 3-Phenylpropyl, Cyclopentyl, Cyclopentylmethyl, 2-Cyclopentylethyl, 3-Cyclopentylpropyl, Cyclohexyl, Cyclohexylmethyl, 2-Cyclohexylethyl oder 3-Cyclohexylpropyl für einen unsubstituierten C₁- bis C₁₂-Alkylrest und beispielsweise 2-Hydroxyethyl oder 2-Hydroxypropyl für einen substituierten C₁- bis C₁₂-Alkylrest;
- einen unsubstituierten oder substituierten, unverzweigten oder verzweigten, acyclischen oder cyclischen C₂- bis C₁₂-Alkenylrest, wie beispielsweise Vinyl (Ethenyl), 2-Propenyl, 1-Methylvinyl, 3-Butenyl, cis-2-Butenyl, trans-2-Butenyl, 3-Cyclopentenyl, 2-Cyclohexenyl, 3-Cyclohexenyl oder 2,5-Cyclohexadienyl;
- einen unsubstituierten oder substituierten aromatischen Rest mit einem Ring oder zwei oder drei kondensierten Ringen, bei dem eines oder mehrere Ringatome durch Heteroatome, wie beispielseise Stickstoff, substituiert sein können und bei dem ein oder mehrere der Wasserstoffatome durch Substituenten, wie beispielsweise Alkyl- oder Arylgruppen ersetzt sein können; oder
- eine oligomere oder polymere Gruppe, wie beispielsweise einem Cellulose-, einem Polyvinylalkohol-, einem Zucker-, einem Zuckeralkohol- oder einem Glycerin-Rest.

Besonders bevorzugt setzt man einen Alkohol (VIc) ein, bei dem der Rest R für einen unsubstituierten, unverzweigten oder verzweigten, acyclischen C₁- bis C₆-Alkylrest, konkret Methyl, Ethyl, Propyl, 1-Methylethyl, Butyl, 1-Methylpropyl, 2-Methylpropyl, 1,1-Dimethylethyl, Pentyl und Hexyl sowie für den substituierten C₂-Alkylrest 2-Hydroxyethyl steht. Ganz besonders bevorzugt setzt man Methanol, Ethanol, Propanol und Butanol ein.

Beim erfindungsgemäßen Verfahren können bei der Umsetzung mit einem Alkohol in Schritt (c) prinzipiell alle geeigneten Verfahren zur Umsetzung von Carbonsäureanhydriden mit Alkoholen eingesetzt werden. Im Allgemeinen führt man die Reaktion in Gegenwart eines Katalysators durch, wobei als Katalysatoren in der Regel jene Katalysatoren eingesetzt werden können, welche auch zur Katalyse der Umesterungsreaktion (Schritt (a)), geeignet sind. Auf die dort beschriebenen Katalysatoren sei hiermit verwiesen.

Die Umsetzung mit einem Alkohol kann auch in Gegenwart eines zusätzlichen inerten Lösungsmittels durchgeführt werden. Als inerte Lösungsmittel sind Lösungsmittel zu verstehen, welche unter den eingestellten Reaktionsbedingungen chemisch nicht mit den eingesetzten Verbindungen, d.h. den Edukten, den Produkten sowie den Katalysatoren, reagieren. Als inerte Lösungsmittel geeignet sind beispielsweise jene Lösungsmittel, welche bei der Umesterung (Schritt (a)) beschrieben sind.

Die Durchführung der Reaktion, die zu wählenden Reaktionsparameter, die Wahl geeigneter Reaktionsapparate und die Aufarbeitung und Trennung des Reaktionsgemisches orientiert sich stark an den Ausführungen zur Hydrolyse, worauf hiermit verwiesen sei.

Die konkret zu wählenden Verfahrensparameter und Maßnahmen sind unter anderem von der Natur des eingesetzten Carbonsäureanhydrids (V), des eingesetzten Alkohols, der gebildeten Reaktionsprodukte und dem ausgewählten Katalysator abhängig und mit dem üblichen Fachkönnen zu ermitteln.

Beim erfindungsgemäßen Verfahren ist der Einsatz des Carbonsäureanhydrids (V) als Acylierungsreagens gegenüber einem Alkohol unter Bildung eines Carbonsäureesters bevorzugt.

Beim Einsatz eines Diols als Alkohol (VIc) bildet sich der entsprechende Dicarbonsäure-diester. Für den Einsatz des bevorzugten 1,2-Ethandiols (Glykol) ergibt sich die folgende Reaktionsgleichung:

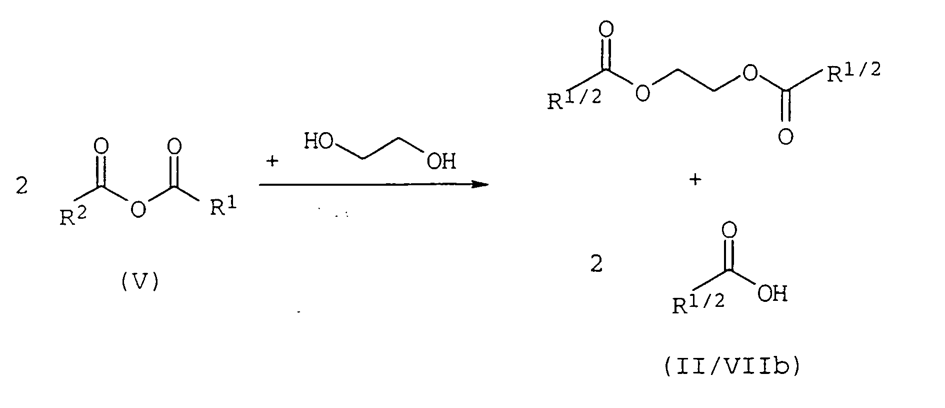

Der gebildete Dicarbonsäure-diester kann beispielsweise in einer Folgestufe zum entsprechenden Carbonsäurevinylester und der Carbonsäure (II/VIIb) gespalten werden:

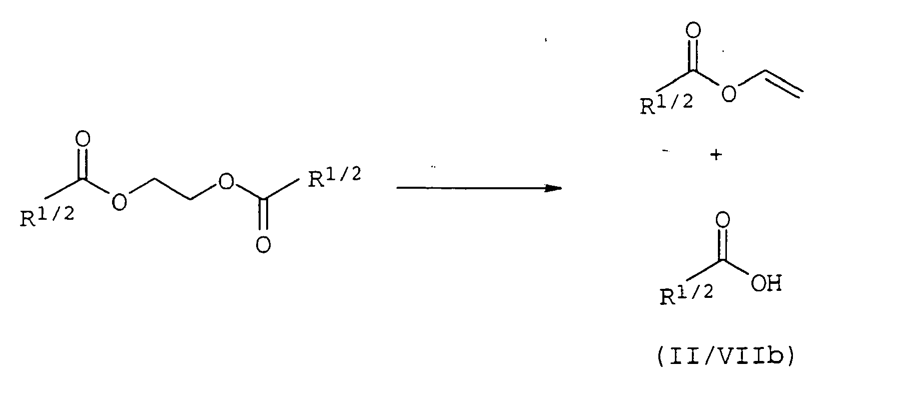

Für die genannte Spaltung können prinzipiell alle geeigneten Verfahren hierzu eingesetzt werden. Sie ist beispielsweise in GB-A 1 365 351 und US 3,787,485 beschrieben und erfolgt im Allgemeinen bei einer Temperatur im Bereich 200 bis 800°C und bevorzugt 400 bis 600°C und einem Druck von 0,01 bis 1,0 MPa abs in der Gasphase in Gegenwart oder Abwesenheit eines Katalysators. Als geeignete Reaktoren für die Umsetzung der zweiten Stufe seien beispielsweise Strömungsrohre genannt. Das gebildete Reaktionsgas, welches den Carbonsäurevinylester, die Carbonsäure (II), nicht-umgesetzten Dicarbonsäure-diester sowie Nebenprodukte wie beispielsweise Kohlendioxid oder Methan enthält, sollte zur Vermeidung der Zersetzung und der Polymerisation des Carbonsäurevinylester möglichst schnell abgekühlt werden. Die Reaktionsprodukte werden im Allgemeinen durch Kondensation und/oder Auswaschung in einem geeigneten Lösungsmittel, bevorzugt der Carbonsäure (II), erhalten. Im Allgemeinen werden in einer nachgeschalteten Stufe weitere Aufarbeitungsschritte, wie etwa Destillation unter erhöhter Temperatur und/oder Vakuum, Kristallisation oder Extraktion, durchgeführt. Die Art und Weise der einzusetzenden Trennverfahren wird in der Regel durch die Eigenschaften der zu trennenden Edukte und Produkte bestimmt. Bei Kenntnis der vorliegenden Edukte, Produkte und gegebenenfalls des Katalysators ist es für den Fachmann ohne Weiteres möglich, ein geeignetes Aufarbeitungskonzept zu entwickeln. Die konkret zu wählenden Verfahrensparameter und Maßnahmen sind mit dem üblichen Fachkönnen zu ermitteln.

### (bb) Die Umsetzung mit Ammoniak oder einem Amin zu einem Carbonsäureamid (VIId)

Exemplarisch sei die Umsetzung des Carbonsäureanhydrids (V) mit einem sekundären Amin dargestellt:

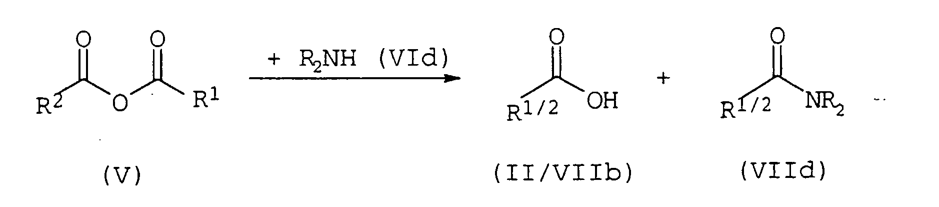

Der Rest R steht im Allgemeinen für Wasserstoff oder einen Kohlenstoff enthaltenden organischen Rest. Bevorzugt steht der Rest R für
- Wasserstoff;
- einen unsubstituierten oder substituierten, unverzweigten oder verzweigten, acyclischen oder cyclischen C₁- bis C₁₂-Alkylrest, wie beispielsweise Methyl, Ethyl, Propyl, 1-Methylethyl, Butyl, 1-Methylpropyl, 2-Methylpropyl, 1,1-Dimethylethyl, Pentyl, Hexyl, Heptyl, 1-Ethylpentyl, Octyl, 2,4,4-Trimethylpentyl, Nonyl, 1,1-Dimethylheptyl, Decyl, Undecyl, Dodecyl, Phenylmethyl, 2-Phenylethyl, 3-Phenylpropyl, Cyclopentyl, Cyclopentylmethyl, 2-Cyclopentylethyl, 3-Cyclopentylpropyl, Cyclohexyl, Cyclohexylmethyl, 2-Cyclohexylethyl, 3-Cyclohexylpropyl oder 2-Aminoethyl;
- einen unsubstituierten oder substituierten, unverzweigten oder verzweigten, acyclischen oder cyclischen C₂- bis C₁₂-Alkenylrest, wie beispielsweise Vinyl (Ethenyl), 2-Propenyl, 1-Methylvinyl, 3-Butenyl, cis-2-Butenyl, trans-2-Butenyl, 3-Cyclopentenyl, 2-Cyclohexenyl, 3-Cyclohexenyl oder 2,5-Cyclohexadienyl;
- einen unsubstituierten oder substituierten aromatischen Rest mit einem Ring oder zwei oder drei kondensierten Ringen, bei dem eines oder mehrere Ringatome durch Heteroatome, wie beispielseise Stickstoff, substituiert sein können und bei dem ein oder mehrere der Wasserstoffatome durch Substituenten, wie beispielsweise Alkyl- oder Arylgruppen ersetzt sein können; oder
- eine oligomere oder polymere Gruppe, wie beispielsweise einem Polyvinylamin- oder einem Polyethylenimin-Rest.

Besonders bevorzugt setzt man eine Verbindung der Formel (VId) ein, bei der die Reste R jeweils für Wasserstoff, einen unsubstituierten, unverzweigten oder verzweigten, acyclischen oder cyclischen C₁- bis C₆-Alkylrest, konkret Methyl, Ethyl, Propyl, 1-Methylethyl, Butyl, 1-Methylpropyl, 2-Methylpropyl, 1,1-Dimethylethyl, Pentyl, Hexyl und Cyclohexyl, oder einem Phenylrest steht. Ganz besonders bevorzugt setzt man Ammoniak, Dimethylamin und Anilin ein.

Die konkret zu wählenden Verfahrensparameter und Maßnahmen sind unter anderem von der Natur des eingesetzten Carbonsäureanhydrids (V), des eingesetzten Amins, der gebildeten Reaktionsprodukte und dem gegebenenfalls gewählten Katalysator abhängig und mit dem üblichen Fachkönnen zu ermitteln.

### (cc) Die Umsetzung mit einem Carbonsäureamid zu einem N-Acyl-carbonsäureamid (VIIe)

Exemplarisch sei die Umsetzung des Carbonsäureanhydrids (V) mit einem primären Carbonsäureamid dargestellt:

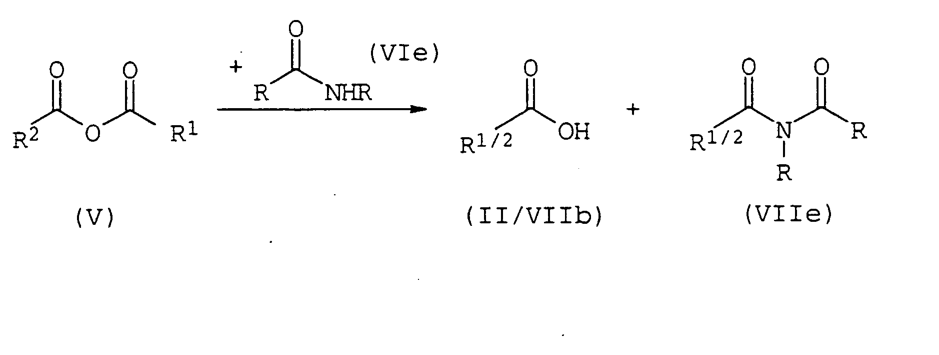

Der Rest R steht im Allgemeinen für Wasserstoff oder einen Kohlenstoff enthaltenden organischen Rest. Bevorzugt hat der Rest R die gleiche Bedeutung wie beim Amin (VId) beschrieben. Ganz besonders bevorzugt setzt man als Amid (VIe) Formamid ein.

Die konkret zu wählenden Verfahrensparameter und Maßnahmen sind unter anderem von der Natur des eingesetzten Carbonsäureanhydrids (V), des eingesetzten Carbonsäureamids, der gebildeten Reaktionsprodukte und dem gegebenenfalls gewählten Katalysator abhängig und mit dem üblichen Fachkönnen zu ermitteln.

### (dd) Die Umsetzung mit einem Aldehyd oder Keton zu einem Acylal oder Acylon und Spaltung zu einem α,β-ungesättigten Carbonsäureester (VIIf)

Die Umsetzung des Carbonsäureanhydrids (V) mit einem Aldehyd zu einem Acylal beziehungsweise mit einem Keton zu einem Acylon und anschließende Spaltung zu einem α,β-ungesättigten Carbonsäureester (VIIf) erfordert den Einsatz eines Aldehyds beziehungsweise Ketons, welcher mindestens ein Wasserstoffatom in α-Stellung zur Carbonyl-Gruppe aufweist. Im Folgenden ist die allgemeine Reaktionsgleichung für den Einsatz eines Aldehyds dargestellt:

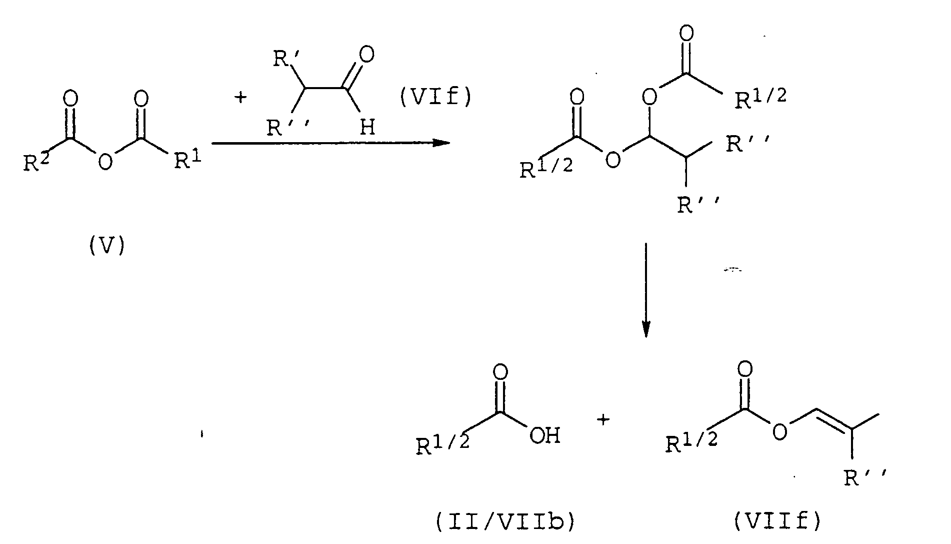

In der allgemeinen Reaktionsgleichtung besitzt der Rest (R')(R")CH im Allgemeinen die gleiche Definition wie die Reste R¹ und R². Bevorzugt steht der Rest (R')(R")CH für
- einen unsubstituierten oder substituierten, unverzweigten oder verzweigten, acyclischen oder cyclischen C₁- bis C₁₂-Alkylrest mit einem Wasserstoffatom in α-Stellung, wie beispielsweise Methyl, Ethyl, Propyl, 1-Methylethyl, Butyl, 1-Methylpropyl, 2-Methylpropyl, Pentyl, Hexyl, Heptyl, 1-Ethylpentyl, Octyl, 2,4,4-Trimethylpentyl, Nonyl, 1,1-Dimethylheptyl, Decyl, Undecyl, Dodecyl, Phenylmethyl, 2-Phenylethyl, 3-Phenylpropyl, Cyclopentyl, Cyclopentylmethyl, 2-Cyclopentylethyl, 3-Cyclopentylpropyl, Cyclohexyl, Cyclohexylmethyl, 2-Cyclohexylethyl oder 3-Cyclohexylpropyl; oder
- einen unsubstituierten oder substituierten, unverzweigten oder verzweigten, acyclischen oder cyclischen C₂- bis C₁₂-Alkenylrest, wie beispielsweise Vinyl (Ethenyl), 2-Propenyl, 1-Methylvinyl, 3-Butenyl, cis-2-Butenyl, trans-2-Butenyl, 3-Cyclopentenyl, 2-Cyclohexenyl, 3-Cyclohexenyl oder 2,5-Cyclohexadienyl.

Besonders bevorzugt setzt man einen Aldehyd (VIf) ein, bei dem der Rest (R')(R")CH für einen unsubstituierten, unverzweigten oder verzweigten, acyclischen C₁- bis C₆-Alkylrest, konkret Methyl, Ethyl, Propyl, 1-Methylethyl, Butyl, 1-Methylpropyl, 2-Methylpropyl, 1,1-Dimethylethyl, Pentyl und Hexyl steht. Ganz besonders bevorzugt setzt man Acetaldehyd, Propionaldehyd und Butyraldehyd und Isobutyraldehyd (2-Methylpropionaldehyd), insbesondere Acetaldehyd ein.

Als Keton (VIf) setzt man bevorzugt Aceton (Propanon) ein.

Alternativ ist es auch möglich, geschützte Aldehyde und Ketone, wie beispielsweise Acetale oder Ketale, einzusetzen, wie beispielsweise in J56 040 642 beschrieben.

Für die Umsetzung des Carbonsäureanhydrids (V) zu einem α,β-ungesättigten Carbonsäureester (VIIf) können prinzipiell alle geeigneten Verfahren hierzu eingesetzt werden. In der Regel erfolgt die Umsetzung in einem zweistufigen Verfahren, wie es beispielsweise in GB-A 2 013 184 beschrieben ist.

In der ersten Stufe setzt man das Carbonsäureanhydrid (V) mit dem Aldehyd (VIf) oder dem Keton kontinuierlich in Gegenwart eines sauren Katalysators im Allgemeinen in der Flüssigphase zum entsprechenden Additionsprodukt um. Als Katalysatoren können in der Regel jene Katalysatoren eingesetzt werden, welche auch zur Katalyse der Umesterungsreaktion (Schritt (a)), geeignet sind. Auf die dort beschriebenen Katalysatoren sei hiermit verwiesen. Im Allgemeinen führt man die erste Stufe der Umsetzung bei einer Temperatur von 50 bis 150°C, bevorzugt von 120 bis 140°C durch. Der Druck in der ersten Stufe der genannten Umsetzung ist in der Regel nicht von Bedeutung. Er sollte jedoch aus praktischen Erwägungen derart eingestellt werden, dass die beiden Edukte (V) und (VIf) sowie das Additionsprodukt überwiegend in der Flüssigphase vorliegen. Um die Bildung des Additionsprodukts zu fördern setzt man in der Regel eine Überschuß an Carbonsäureanhydrid (V) ein. Im Allgemeinen beträgt das molare Verhältnis von (V):(VIf) im Reaktionsgemisch 1 bis 40. Als geeignete Reaktoren für die Umsetzung der ersten Stufe seien beispielsweise Rührkesselreaktoren, Strömungsrohre, Schachttreaktoren oder Strahlschlaufenreaktoren genannt.

Alternativ kann die erste Stufe der Umsetzung zur Bildung des Additionsprodukts auch in der Gasphase in Gegenwart eines heterogenen Katalysators durchgeführt werden.

Aus der ersten Stufe entnimmt man die Reaktionslösung im Allgemeinen kontinuierlich und führt sie der zweiten Stufe zur Spaltung in die Carbonsäure (II/VIIb) und dem α,β-ungesättigten Carbonsäureester (VIIf) zu. Die Spaltung ist beispielsweise in GB-A 2 013 184 und EP-A 0 348 309 beschrieben. Sie erfolgt in der Flüssigphase in Gegenwart eines sauren Katalysators, wobei in der Regel jene Katalysatoren eingesetzt werden können, welche auch zur Katalyse der Umesterungsreaktion (Schritt (a)), geeignet sind. Auf die dort beschriebenen Katalysatoren sei hiermit verwiesen. Im Allgemeinen führt man die zweite Stufe der Umsetzung bei einer Temperatur von 60 bis 200°C, bevorzugt von 100 bis 140°C durch. Auch der Druck in der zweiten Stufe der genannten Umsetzung ist in der Regel nicht von Bedeutung. Im Allgemeinen liegt dieser im Bereich von 0,05 bis 1 MPa abs. Auch die Spaltung wird in der Regel in Gegenwart eines Überschußes an Carbonsäureanhydrid (V), wie für die erste Stufe beschrieben, durchgeführt. Als geeignete Reaktoren für die Umsetzung der zweiten Stufe seien beispielsweise Rührkesselreaktoren, Strömungsrohre, Schachttreaktoren oder Strahlschlaufenreaktoren genannt.

Beide Verfahrensstufen können auch in Gegenwart eines zusätzlichen inerten Lösungsmittels durchgeführt werden. Als inerte Lösungsmittel sind Lösungsmittel zu verstehen, welche unter den eingestellten Reaktionsbedingungen chemisch nicht mit den eingesetzten Verbindungen, d.h. den Edukten, den Produkten sowie den Katalysatoren, reagieren. Als inerte Lösungsmittel geeignet sind beispielsweise jene Lösungsmittel, welche bei der Umesterung (Schritt (a)) beschrieben sind.

Bevorzugt werden die Reaktionsbedingungen in der zweiten Stufe derart eingestellt, dass eine Verdampfung der Produkte, der nicht-umgesetzten Edukte, der Nebenprodukte und/oder des gegebenenfalls eingesetzten Lösungsmittels möglich ist. Die verdampften Komponenten werden anschließend der weiteren Aufarbeitung, welche bevorzugt ebenfalls destillativ erfolgt, zugeführt. Die verbleibende Lösung, welche den Katalysator enthält, wird in die erste Stufe rückgeführt. Ist eine Verdampfung der beiden Produkte Carbonsäure (II) und α,β-ungesättigter Carbonsäureester (VIIf) im Reaktor der zweiten Stufe nicht möglich, so werden diese im Allgemeinenen in einer nachgeschalteten Stufe durch geeignete Aufarbeitungsschritte, wie etwa Destillation unter erhöhter Temperatur und/oder Vakuum, Kristallisation oder Extraktion, abgetrennt. Die Art und Weise der einzusetzenden Trennverfahren wird in der Regel durch die Eigenschaften der zu trennenden Edukte und Produkte bestimmt. Bei Kenntnis der vorliegenden Edukte, Produkte und gegebenenfalls des Katalysators ist es für den Fachmann ohne Weiteres möglich, ein geeignetes Aufarbeitungskonzept zu entwickeln.

Die konkret zu wählenden Verfahrensparameter und Maßnahmen sind unter anderem von der Natur des eingesetzten Carbonsäureanhydrids (V), des eingesetzten Aldehyds oder Ketons, der gebildeten Reaktionsprodukte und dem ausgewählten Katalysator abhängig und mit dem üblichen Fachkönnen zu ermitteln.

Beim erfindungsgemäßen Verfahren ist der Einsatz des Carbonsäureanhydrids (V) als Acylierungsreagens gegenüber einem Aldehyd oder Keton unter Bildung eines α,β-ungesättigten Carbonsäureesters bevorzugt.

### (ee) Die Umsetzung mit einem aromatischen Kohlenwasserstoff zu einem aromatischen Keton (VIIg)

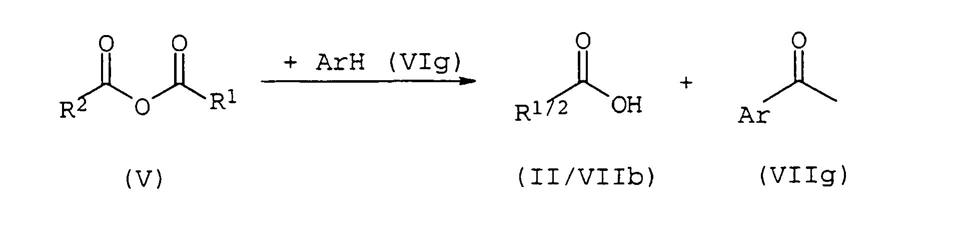

In der allgemeinen Reaktionsgleichung steht der Rest Ar für einen unsubstituierten oder substituierten, gegebenenfalls heterocyclischen aromatischen Rest mit bevorzugt ein bis drei aromatischen Ringen. Besonders bevorzugt setzt man als aromatischen Kohlenwasserstoff Benzol, Toluol oder Xylole ein.

Beim erfindungsgemäßen Verfahren können prinzipiell alle geeigneten Verfahren zur Acetylierung aromatischer Kohlenwasserstoffe mit Carbonsäureanhydriden eingesetzt werden. Die konkret zu wählenden Verfahrensparameter und Maßnahmen der Synthesestufe und der anschließenden Aufarbeitung und Trennung sind unter anderem von der Natur des eingesetzten Carbonsäureanhydrids (V), des eingesetzten aromatischen Kohlenwasserstoffs, der gebildeten Reaktionsprodukte und dem ausgewählten Katalysator abhängig und mit dem üblichen Fachkönnen zu ermitteln.

### (iv) Hydrierung

Bei der Hydrierung des Carbonsäureanhydrids (V) bildet sich der entsprechende Aldehyd und die Carbonsäure (II).

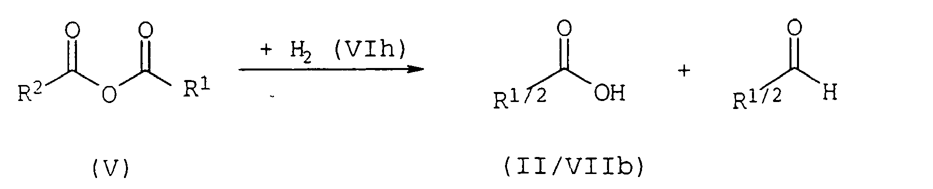

Beim erfindungsgemäßen Verfahren können bei der Hydrierung in Schritt (c) prinzipiell alle geeigneten Verfahren zur Hydrierung von Carbonsäureanhydriden eingesetzt werden.

Besitzt der Rest "R1/2" des gebildeten Aldehyds ein Wasserstoffatom in α-Stellung zur Carbonyl-Gruppe, so bleibt die Reaktion im Allgemeinen nicht auf der Stufe des Aldehyds stehen, da sich dieser bevorzugt mit weiterem Carbonsäureanhydrid zu einem Acylal umsetzt. Unter geeigneten Reaktionsbedingungen setzt sich dieses unter Spaltung zu einem α,β-ungesättigten Carbonsäureester und der entsprechenden Carbonsäure (II/VIIb) um.

Verfahren hierzu sind bekannt und beispielsweise in US 4,978,778 beschrieben, worauf hiermit explizit Bezug genommen wird. Im Allgemeinen setzt man hierzu ein Katalysatorsystem ein, welches ein Metall der 8. bis 10. Gruppe des Periodensystems, bevorzugt Palladium, Rhodium, Ruthenium, Platin, Osmium, Cobalt oder Nickel, eine Protonen- oder Lewis-Säure, bevorzugt eine Säure, wie sie auch für die Umesterungsreaktion in Schritt (a) geeignet ist, und eine halogenhaltige Verbindung, bevorzugt ein Halogenmethan, enthält. Im Allgemeinen führt man die Umsetzung bei einer Temperatur von 50 bis 250°C und einem Wasserstoffpartialdruck von 0,02 bis 10 MPa durch. In der Regel ist es von Vorteil, die Umsetzung in Gegenwart von Kohlenmonoxid durchzuführen, da dieses im Allgemeinen die Katalysatorstabilität erhöht und die Selektivität verbessert. Die konkret zu wählenden Verfahrensparameter und Maßnahmen sind unter anderem von der Natur des eingesetzten Carbonsäureanhydrids (V) der gebildeten Reaktionsprodukte und dem ausgewählten Katalysator abhängig und mit dem üblichen Fachkönnen zu ermitteln.

In einer bevorzugten Variante des erfindungsgemäßen Verfahrens, bei der das Carbonsäureanhydrid (V) durch Hydrierung in die Carbonsäure (II/VIIb) überführt wird, führt man die Carbonylierung von Schritt (b) und die Hydrierung von Schritt (c) zusammen in einem Reaktionsapparat durch. Ein geeignetes Verfahren ist beispielsweise in EP-A 0 048 174 beschrieben, worauf hiermit explizit Bezug genommen wird. Im Allgemeinen setzt man hierzu ein Katalysatorsystem ein, welches ein Metall der 8. bis 10. Gruppe des Periodensystems, bevorzugt Palladium, Rhodium, Ruthenium, Platin, Osmium, Cobalt oder Nickel, eine halogenhaltige Verbindung, bevorzugt ein Halogenmethan, einen Stickstoff- oder Phosphor-enthaltenden.Promotor, bevorzugt ein aliphatisches oder aromatisches Amin oder ein Phosphin und gegebenenfalls eine Protonen- oder Lewis-Säure, bevorzugt eine Säure, wie sie auch für die Umesterungsreaktion in Schritt (a) geeignet ist, enthält. Im Allgemeinen führt man die Umsetzung bei einer Temperatur von 80 bis 350°C und bevorzugt von 100 bis 250°C und einem Wasserstoffpartialdruck von 0,3 bis 30 MPa durch. Im Allgemeinen führt man Kohlenmonoxid und Wasserstoff in einem molaren CO:H₂-Verhältnis von 0,5 bis 5 zu. Die konkret zu wählenden Verfahrensparameter und Maßnahmen sind unter anderem von der Natur des eingesetzten Carbonsäureanhydrids (V) der gebildeten Reaktionsprodukte und dem ausgewählten Katalysator abhängig und mit dem üblichen Fachkönnen zu ermitteln.

In einer bevorzugten Ausführungsform des erfindungsgemäßen Verfahrens setzt man mindestens einen Teil der in Schritt (c) gebildeten Carbonsäure (II) in Schritt (a) ein. Bevorzugt führt man mindestens 10%, besonders bevorzugt mindestens 50% und ganz besonders bevorzugt mindestens 90% der Carbonsäure (II), welche in Schritt (a) für die Umesterung erforderlich ist, aus Schritt (c) zu. Insbesondere führt man die gesamte Menge an Carbonsäure (II), welche in Schritt (a) für die Umesterung erforderlich ist, aus Schritt (c) zu. Um dies zu gewährleisten, ist die Produktabführung, d.h. die Produktentnahme aus dem erfindungsgemäßen Kreisprozeß, derart zu gestalten, dass bei Schritt (c) mindestens soviel Carbonsäure (II) gebildet wird, wie in Schritt (a) als rückgeführte Carbonsäure (II) eingesetzt werden soll.

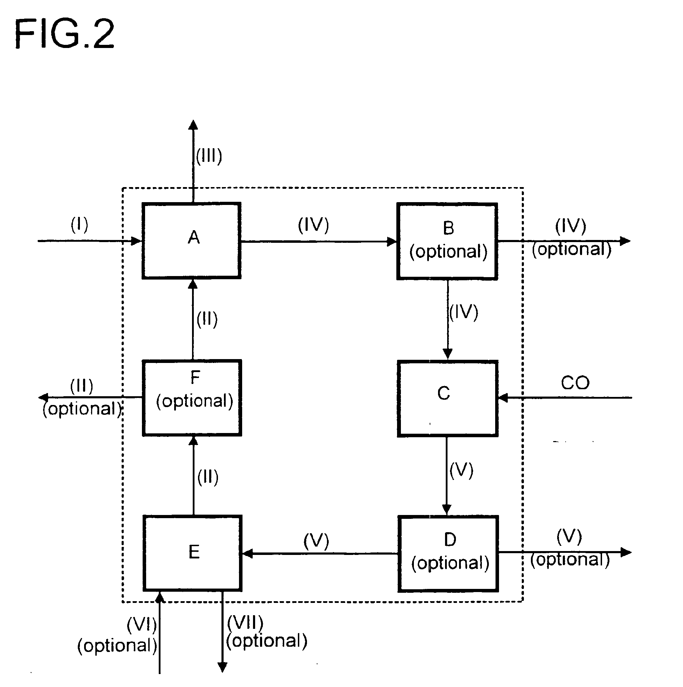

Abbildung 2 zeigt ein Blockdiagramm des bevorzugten erfindungsgemäßen Verfahrens. Für die Blöcke "A" bis "C" sei auf die Beschreibung des Blockdiagramms von Abbildung 1 verwiesen. Das aus Block "C" (Carbonylierung) stammende Carbonsäureanhydrid (V) wird über einen optional vorhandenen Block "D" (Ausschleusung Carbonsäureanhydrid), bei dem gegebenenfalls ein Teil des gebildeten Carbonsäureanhydrids (V) als Endprodukt ausgeschleust werden kann, dem Block "E" (Überführung in die Carbonsäure (II)/Trennung) zugeführt. Abhängig von der Art der gewählten Überführungsreaktion ist die Zufuhr eines Edukts (VI) zu Block "E" erforderlich oder nicht. Als Reaktionsprodukt/e wird/werden in Block "E" die Carbonsäure (II) und je nach Art der gewählten Überführungsreaktion noch ein weiteres Produkt (VII) gebildet. Das optional gebildete Produkt (VII) wird abgetrennt und als Endprodukt ausgeschleust. Die Carbonsäure (II) wird über einen optional vorhandenen Block "F" (Ausschleusung Carbonsäure), bei dem gegebenenfalls ein Teil der gebildeten Carbonsäure (II) als Endprodukt ausgeschleust werden kann, dem Block "A" (Umesterung/Trennung) zugeführt.

Beim erfindungsgemäßen Verfahren setzt man bevozugt einen Ameisensäureester (I) 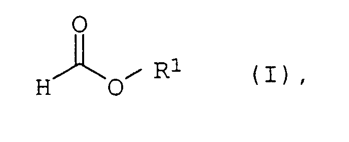 ein, bei dem der Rest R¹ für
- einen unsubstituierten oder substituierten, unverzweigten oder verzweigten, acyclischen oder cyclischen C₁- bis C₁₂-Alkylrest, wie beispielsweise Methyl, Ethyl, Propyl, 1-Methylethyl, Butyl, 1-Methylpropyl, 2-Methylpropyl, 1,1-Dimethylethyl, Pentyl, Hexyl, Heptyl, 1-Ethylpentyl, Octyl, 2,4,4-Trimethylpentyl, Nonyl, 1,1-Dimethylheptyl, Decyl, Undecyl, Dodecyl, Phenylmethyl, 2-Phenylethyl, 3-Phenylpropyl, Cyclopentyl, Cyclopentylmethyl, 2-Cyclopentylethyl, 3-Cyclopentylpropyl, Cyclohexyl, Cyclohexylmethyl, 2-Cyclohexylethyl oder 3-Cyclohexylpropyl; oder
- einen unsubstituierten oder substituierten, unverzweigten oder verzweigten, acyclischen oder cyclischen C₂- bis C₁₂-Alkenylrest, wie beispielsweise Vinyl (Ethenyl), 2-Propenyl, 1-Methylvinyl, 3-Butenyl, cis-2-Butenyl, trans-2-Butenyl, 3-Cyclopentenyl, 2-Cyclohexenyl, 3-Cyclohexenyl oder 2,5-Cyclohexadienyl;
steht.

Besonders bevorzugt setzt man einen Ameisensäureester (I) ein, bei dem der Rest R¹ für einen unsubstituierten, unverzweigten oder verzweigten, acyclischen C₁- bis C₆-Alkylrest, konkret Methyl, Ethyl, Propyl, 1-Methylethyl, Butyl, 1-Methylpropyl, 2-Methylpropyl, 1,1-Dimethylethyl, Pentyl und Hexyl steht. Ganz besonders bevorzugt setzt man Ameisensäuremethylester, Ameisensäureethylester, Ameisensäurepropylester und Ameisensäurebutylester und insbesondere Ameisensäuremethylester ein.

Beim erfindungsgemäßen Verfahren setzt man bevozugt eine Carbonsäure (II) 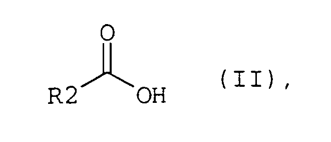 ein, bei der der Rest R² für
- einen unsubstituierten oder substituierten, unverzweigten oder verzweigten, acyclischen oder cyclischen C₁- bis C₁₂-Alkylrest, wie beispielsweise Methyl, Ethyl, Propyl, 1-Methylethyl, Butyl, 1-Methylpropyl, 2-Methylpropyl, 1,1-Dimethylethyl, Pentyl, Hexyl, Heptyl, 1-Ethylpentyl, Octyl, 2,4,4-Trimethylpentyl, Nonyl, 1,1-Dimethylheptyl, Decyl, Undecyl, Dodecyl, Phenylmethyl, 2-Phenylethyl, 3-Phenylpropyl, Cyclopentyl, Cyclopentylmethyl, 2-Cyclopentylethyl, 3-Cyclopentylpropyl, Cyclohexyl, Cyclohexylmethyl, 2-Cyclohexylethyl, 3-Cyclohexylpropyl, Chlormethyl, Dichlormethyl, Trichlormethyl; oder
- einen unsubstituierten oder substituierten, unverzweigten oder verzweigten, acyclischen oder cyclischen C₂- bis C₁₂-Alkenylrest, wie beispielsweise Vinyl (Ethenyl), 2-Propenyl, 1-Methylvinyl, 3-Butenyl, cis-2-Butenyl, trans-2-Butenyl, 3-Cyclopentenyl, 2-Cyclohexenyl, 3-Cyclohexenyl oder 2,5-Cyclohexadienyl;
steht.

Besonders bevorzugt setzt man eine Carbonsäure (11) ein, bei der der Rest R² für
- einen unsubstituierten oder substituierten, unverzweigten oder verzweigten, acyclischen C₁- bis C₆-Alkylrest, konkret Methyl, Ethyl, Propyl, 1-Methylethyl, Butyl, 1-Methylpropyl, 2-Methylpropyl, 1,1-Dimethylethyl, Pentyl, Hexyl, Chlormethyl oder Dichlormethyl, Trichlormethyl; oder
- einen unsubstituierten, unverzweigten oder verzweigten, acyclischen C₂- bis C₆-Alkenylrest, wie beispielsweise Vinyl (Ethenyl), 2-Propenyl, 1-Methylvinyl, 3-Butenyl, cis-2-Butenyl oder trans-2-Butenyl;
steht. Ganz besonders bevorzugt setzt man Essigsäure und Propionsäure, insbesondere Essigsäure ein.

Besonders bevorzugt stellt man beim erfindungsgemäßen Verfahren Ameisensäure sowie Essigsäuremethylester, Essigsäureanhydrid, Essigsäure, Keten, Essigsäurevinylester, Essigsäureethylester, Essigsäurepropylester und/oder Essigsäurebutylester her.

Beim erfindungsgemäßen Verfahren setzt man bei der Umesterung in Schritt (a) den Ameisensäureester (I) und die Carbonsäure (II) im Allgemeinen in einem Verhältnis von 1:1 ein, wobei die relativen Konzentrationen im Reaktionsgemisch gegebenenfalls davon abweichen können. Die Carbonsäure (II) wird dabei als Edukt, als Rückführungsstrom aus Schritt (c) oder als Mischform von beiden zugeführt. Pro Mol an umgesetztem Ameisensäureester (I) sowie an umgesetzter Carbonsäure (II) ensteht entsprechend der Reaktionsgleichung 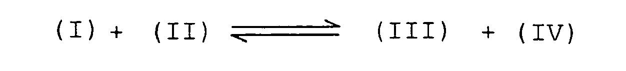 ein Mol Ameisensäure (III) als abzuführendes Produkt sowie ein Mol Carbonsäureester (IV). Da mindestens ein Teil des in Schritt (a) gebildeten Carbonsäureesters (IV) zum entsprechenden Carbonsäureanhydrid (V) carbonyliert wird, werden somit entsprechend der Reaktionsgleichung 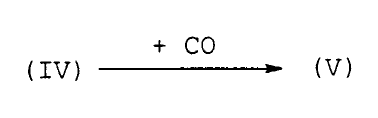 in Summe ein Mol an Carbonsäureesters (IV) und Carbonsäureanhydrid (V) als abzuführendes Produkt gebildet.

Wird entsprechend dem bevorzugten Verfahren mindestens ein Teil des in Schritt (b) gebildeten Carbonsäureanhydrids (V) in die Carbonsäure (II) überführt, so werden entsprechend der Reaktionsgleichung 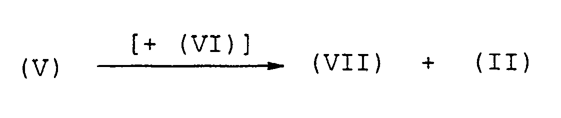 in Summe ein Mol an Carbonsäureester (IV), Carbonsäureanhydrid (V) und Carbonsäurederivat (VII) als abzuführendes Produkt gebildet. Für den Fall der Hydrolyse eines symmetrischen Carbonsäureanhydrids (V) werden pro Mol Carbonsäureanhydrids (V) zwei Mol an Carbonsäure (II) gebildet

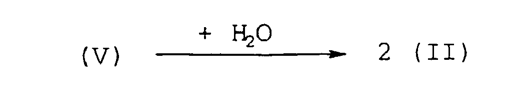

Die gebildete Carbonsäure (II) kann als Produkt abgeführt oder der Umesterung in Schritt (a) wieder zugeführt werden.

Bei entsprechender Wahl der abzuführenden Produktmengen ist es problemlos möglich, das bevorzugte Verfahren mit Rückführung der Carbonsäure (II) so zu gestalten, dass nahezu die gesamte Menge an erforderlicher Carbonsäure (II) für Schritt (a) aus der Rückführung stammt. Geringfügige Verluste, beispielsweise durch Selektivitäten kleiner 100% oder unerwünschtem Austrag, können durch entsprechende Zugaben an Carbonsäure (II) ausgeglichen werden. Wird bei dem durchgeführten Verfahren ein symmetrisches Carbonsäureanhydrid (V) gebildet, wie es beispielsweise bei dem ganz besonders bevorzugten Einsatz von Ameisensäuremethylester (I) und Essigsäure (II) der Fall ist, so können die geringfügigen Verluste durch Hydrolyse des Carbonsäureanhydrids (V) entsprechend der obigen Reaktionsgleichung ausgeglichen werden. Tabelle 1 enthält eine Übersicht der bevorzugten Verfahrensvarianten unter Berücksichtigung der stöchiometrischen Verhältnisse, wobei die gebildete Ameisensäure (III) als Bezugsgröße verwendet wurde. Die letzte Spalte enthält die erforderlichen Verfahrensblöcke, wobei der Übersichtlichkeit halber auf die Nennung der optionalen Blöcke zur Ausschleusung möglicher Zwischenprodukte verzichtet wurde.

Im Folgenden seien, ohne limitierend zu wirken, einige bevorzugte Ausführungsformen näher erläutert.

Ausführungsform 1: Herstellung von Ameisensäure und Essigsäureanhydrid

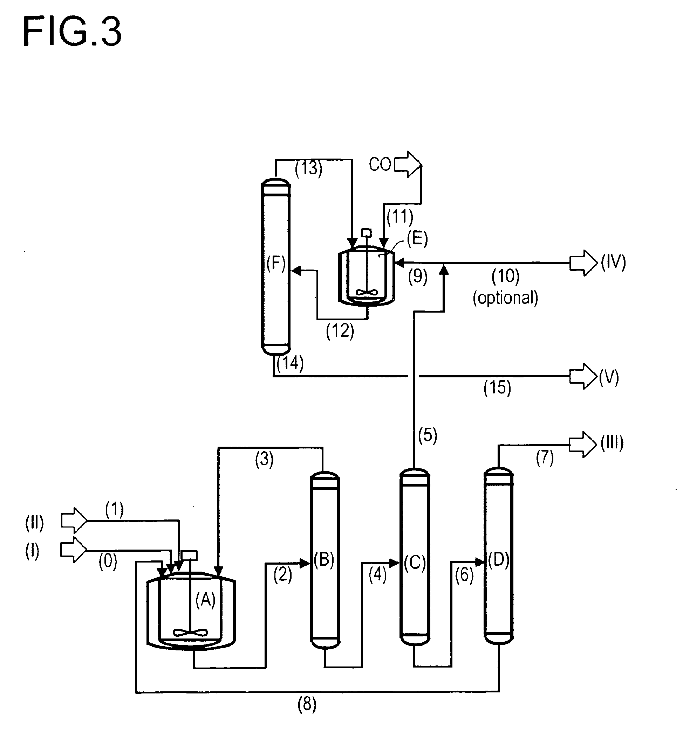

Ein vereinfachtes Verfahrensfließbild ist in Abbildung 3 dargestellt. Ameisensäuremethylester (I) und Essigsäure (II) werden dem Reaktor (A), welcher exemplarisch als Rührkessel dargestellt ist, über Leitung (0) und (1) kontinuierlich zugeführt. Als Reaktor (A) können hierfür jedoch auch andere geeignete Reaktionsapparate, wie beispielsweise weiter oben unter Schritt (a) beschrieben, eingesetzt werden. Im Reaktor (A) findet in Gegenwart des eingesetzten Katalysators die Umesterung zur Ameisensäure (III) und dem Essigsäuremethylester (IV) statt. Das Reaktionsgemisch, welches Ameisensäuremethylester (I), Essigsäure (II), Ameisensäure (III), Essigsäuremethylester (IV) sowie den eingesetzten Katalysator enthält, wird kontinuierlich dem Reaktor (A) entnommen und über Leitung (2) der destillativen Aufarbeitung, welche in Form der Kolonnen (B), (C) und (D) exemplarisch dargestellt ist, zugeführt. Über Leitung (3) werden nicht-umgesetzter Ameisensäuremethylester (I) und eventuell gebildete Leichtsieder dem Reaktor (A) rückgeführt. Ameisensäure (III) wird über Leitung (7) entnommen. Über Leitung 8 werden nicht-umgesetzte Essigsäure (II), Katalysator und eventuell gebildete Hochsieder dem Reaktor (A) rückgeführt. Es versteht sich von selbst, dass je nach Bedarf ein Teil des Stroms (8) zur Vermeidung einer Aufpegelung von Hochsiedern kontinuierlich oder diskontinuierlich ausgeschleust und gegebenenfalls weiter aufgearbeitet werden kann. Essigsäuremethylester (IV) wird über Leitung (5) weitergeleitet.

Im Allgemeinen ist es von Vorteil, für die beiden Kolonnen (B) und (C) eine Trennwandkolonne einzusetzen. Strom (3) wird dabei als Kopfstrom, Strom (5) als Seitenstrom und Strom (6) als Sumpfstrom abgeführt.

Über die optionale Leitung (10) ist gegebenenfalls eine Ausschleusung von Essigsäuremethylester (IV) möglich.

Essigsäuremethylester (IV) wird über Leitung (9) dem Reaktor (E), welcher exemplarisch als Rührkessel dargestellt ist, zur Carbonylierung kontinuierlich zugeführt. Als Reaktor (E) können hierfür jedoch auch andere geeignete Reaktionsapparate, wie beispielsweise weiter oben unter Schritt (b) beschrieben, eingesetzt werden. Im Reaktor (E) findet in Gegenwart des eingesetzten Katalysators die Carbonylierung unter Zufuhr von Kohlenmonoxid über Leitung (11) zum Essigsäureanhydrid (V) statt. Das Reaktionsgemisch, welches nicht-umgesetzten Essigsäuremethylester (IV), Essigsäureanhydrid (V) sowie den eingesetzten Katalysator enthält, wird kontinuierlich dem Reaktor (E) entnommen, im Allgemeinen vom Katalysator befreit, beispielsweise in einem Flashverdampfer (der Übersichtlichkeit halber nicht dargestellt), und über Leitung (12) der destillativen Aufarbeitung, welche in Form der Kolonne (F) exemplarisch dargestellt ist, zugeführt. Über Leitung (13) werden nicht-umgesetzter Essigsäuremethylester (IV) und eventuell gebildete Leichtsieder dem Reaktor (E) rückgeführt. Das Sumpfprodukt der Kolonne (F), welches Essigsäureanhydrid (V) sowie eventuell gebildete Hochsieder enthält, wird über Leitung (14) entnommen und im Allgemeinen in einer weiteren Kolonne (der Übersichtlichkeit halber nicht dargestellt) in Essigsäureanhydrid (V) und Hochsieder getrennt. Der Katalysator enthaltende Strom 5 wird in der Regel wieder dem Reaktor (E) rückgeführt. Es versteht sich von selbst, dass je nach Bedarf ein Teil des Hochsieder enthaltenden Stroms zur Vermeidung einer Aufpegelung von Hochsiedern kontinuierlich oder diskontinuierlich ausgeschleust und gegebenenfalls weiter aufgearbeitet werden kann.

Enthält der Strom (12) zudem noch Essigsäure, beispielsweise durch eine partielle Hydrolyse infolge der Zufuhr von Wasser, so ist im Allgemeinen eine weitere Kolonne zur Abtrennung der Essigsäure erforderlich.

Ausführungsform 2: Herstellung von Ameisensäure und Essigsäuremethylester (mit Essigsäurekreislauf)

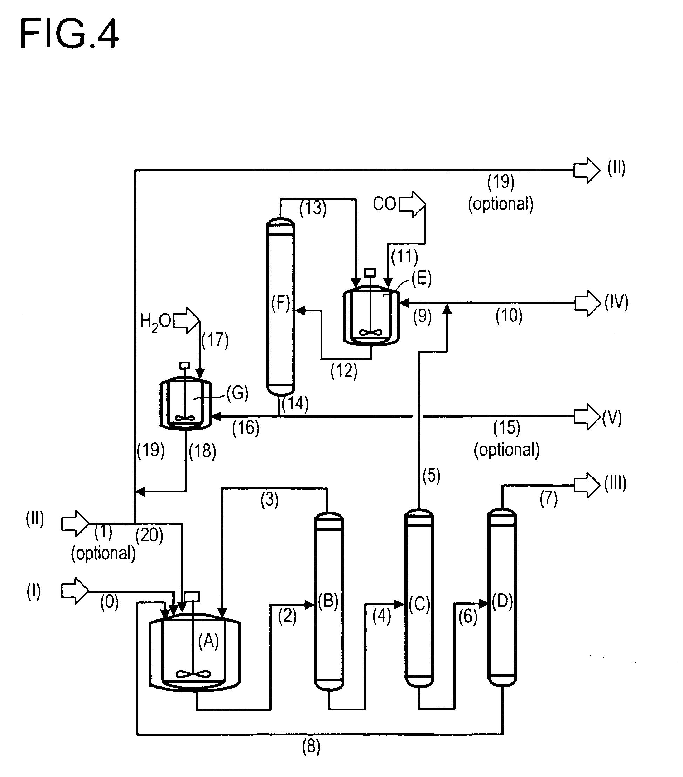

Ein vereinfachtes Verfahrensfließbild ist in Abbildung 4 dargestellt. Die dem Reaktor (A) über Leitung (20) zugeführte Essigsäure (II) stammt zum überwiedenden Teil, bevorzugt vollständig, aus dem Essigsäurekreislauf. Bei Bedarf ist jedoch auch eine Zugabe von zusätzlicher Essigsäure über Leitung (1) möglich. Die Umesterung wird wie in Ausführungsform 1 beschrieben durchgeführt, worauf explizit verwiesen sei.

Über Leitung (10) wird ein Teil des gebildeten Essigsäuremethylesters (IV) entnommen. Der andere Teil wird über Leitung (9) der Carbonylierung zugeführt. Die Carbonylierung wird wie in Ausführungsform 1 beschrieben durchgeführt, worauf explizit verwiesen sei. Über Leitung (15) ist gegebenenfalls eine Ausschleusung von Carbonsäureanhydrid (V) möglich.

Carbonsäureanhydrid (V) wird über Leitung (16) Reaktor (G), welcher exemplarisch als Rührkessel dargestellt ist, zur Hydrolyse zugeführt. Als Reaktor (G) können hierfür jedoch auch andere geeignete Reaktionsapparate, wie beispielsweise weiter oben unter Schritt (c), Hydrolyse (ii) beschrieben, eingesetzt werden. Im Reaktor (G) findet in Gegenwart des eingesetzten Katalysators die Hydrolyse unter Zufuhr von Wasser (VIb) über Leitung (17) zur Essigsäure (II) statt. Das Reaktionsgemisch, welches Essigsäure (II) und den Katalysator enthält, wird kontinuierlich dem Reaktor (G) entnommen und im Allgemeinen in einer weiteren Kolonne (der Übersichtlichkeit halber nicht dargestellt) vom Katalysator befreit.

Über die optionale Leitung (19) ist gegebenenfalls eine Ausschleusung von Essigsäure (II) möglich.

Die Essigsäure (II) wird über Leitung (20) wieder dem Reaktor (A) zugeführt, womit sich der Kreislauf schließt.

Ausführungsform 3: Herstellung von Ameisensäure und Essigsäureanhydrid (mit Essigsäurekreislauf)

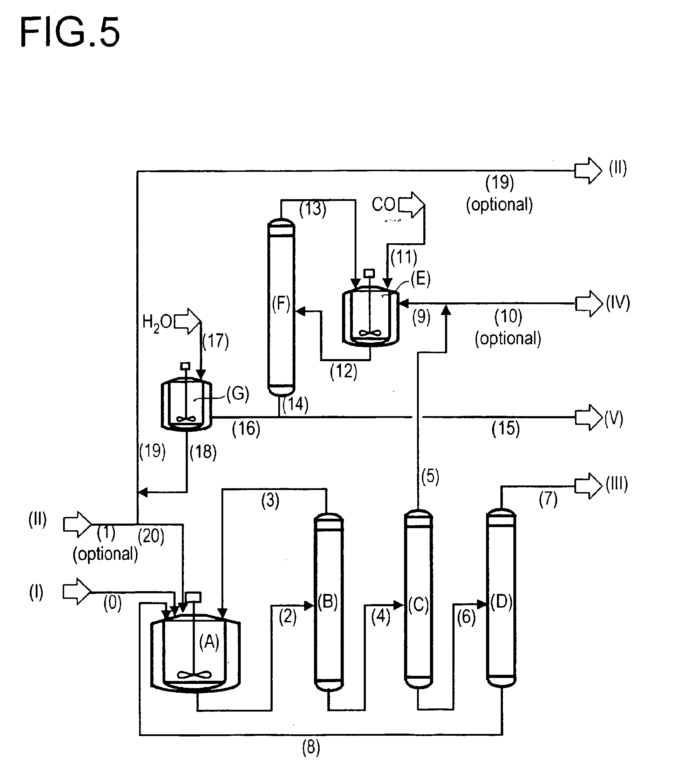

Ein vereinfachtes Verfahrensfließbild ist in Abbildung 5 dargestellt. Das vereinfachte Verfahrensfließbild entspricht im Wesentlichen dem der Ausführungsform 2, wobei ein Teil des gebildeteten Essigsäureanhydrids (V) über Leitung (15) entnommen wird und die Ausschleusung von Essigsäuremethylester (IV) über Leitung (10) optional erfolgt. Der andere Teil des Essigsäureanhydrids (V) wird in Reaktor (G) hydrolisiert und über Leitung (20) wieder dem Reaktor (A) zugeführt, womit sich der Kreislauf schließt.

Ausführungsform 4: Herstellung von Ameisensäure und Essigsäure (mit Essigsäurekreislauf)

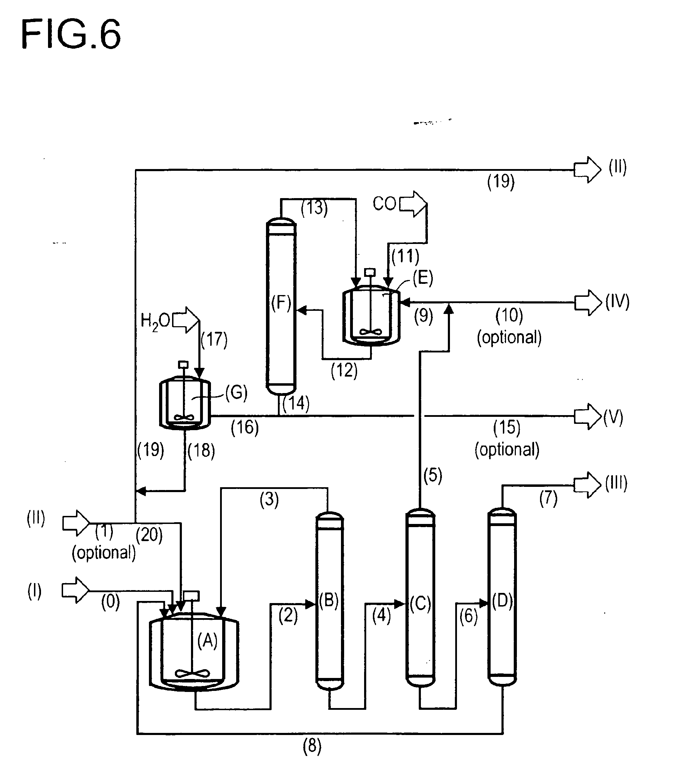

Ein vereinfachtes Verfahrensfließbild ist in Abbildung 6 dargestellt. Das vereinfachte Verfahrensfließbild entspricht im Wesentlichen dem der Ausführungsform 2, wobei ein Teil der gebildeteten Essigsäure (II) über Leitung (19) entnommen wird und die Ausschleusung von Essigsäuremethylester (IV) über Leitung (10) optional erfolgt. Der andere Teil der Essigsäure (II) wird über Leitung (20) wieder dem Reaktor (A) zugeführt, womit sich der Kreislauf schließt.

Ausführungsform 5: Herstellung von Ameisensäure und Keten (mit Essigsäurekreislauf)

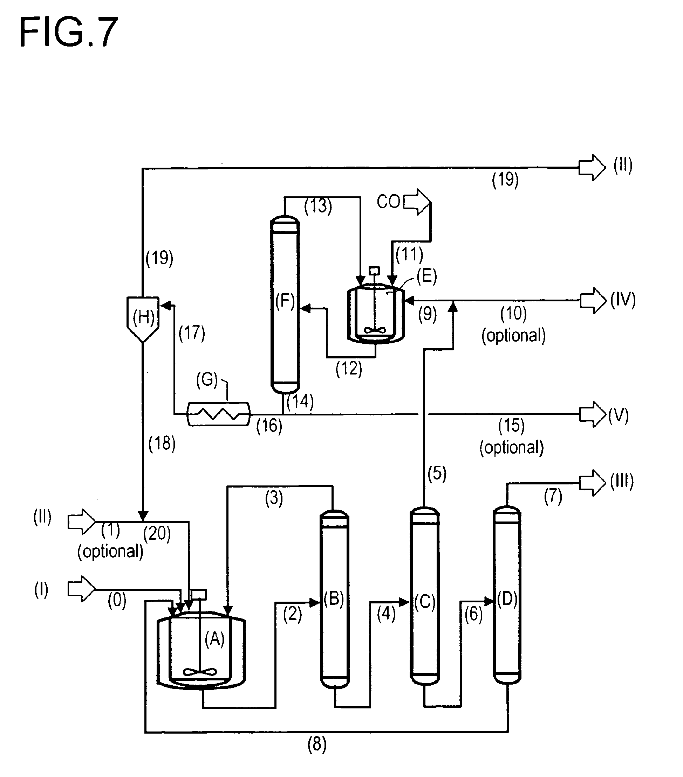

Ein vereinfachtes Verfahrensfließbild ist in Abbildung 7 dargestellt. Der Teil des vereinfachten Verfahrensfließbildes zur Umesterung und zur Carbonylierung entspricht dem in der Ausführungsform 4 dargestelltem Teil. Ein Teil des gebildeteten Essigsäureanhydrids (V) wird über Leitung (16) dem Reaktor (G) zur thermischen Zersetzung zugeführt. Als Reaktor (G) können alle geeigneten Reaktionsapparate, wie beispielsweise weiter oben unter-Schritt (c), thermische Zersetzung (i) beschrieben, eingesetzt werden. Das gebildete Reaktionsgas, welches Keten (VIIa), Essigsäure (II) und nicht-umgesetztes Essigsäureanhydrid (V) enthält, wird über Leitung (17) zur Kondensation und/oder Auswaschung dem Apparat (H) zugeführt. Keten (VIIa) verläßt Apparat (H) gasförmig über Leitung (19). Der kondensierte Strom wird über Leitung (18) entnommen und beispielsweise in einer nachgeschalteten Kolonne (der Übersichtlichkeit halber nicht dargestellt) in Essigsäure (II) und Essigsäureanhydrid (V), welches gegebenenfalls zu Reaktor (G) rückgeführt wird, aufgetrennt oder in einer nachgeschalteten Hydrolysezone vollständig zu Essigsäure (II) hydrolysiert. Die gebildete Essigsäure (II) wird über Leitung (18) und (20) wieder dem Reaktor (A) zugeführt, womit sich der Kreislauf schließt.

Ausführungsform 6: Herstellung von Ameisensäure und Essigsäure-C₁-bis C₄-alkylester (mit Essigsäurekreislauf)

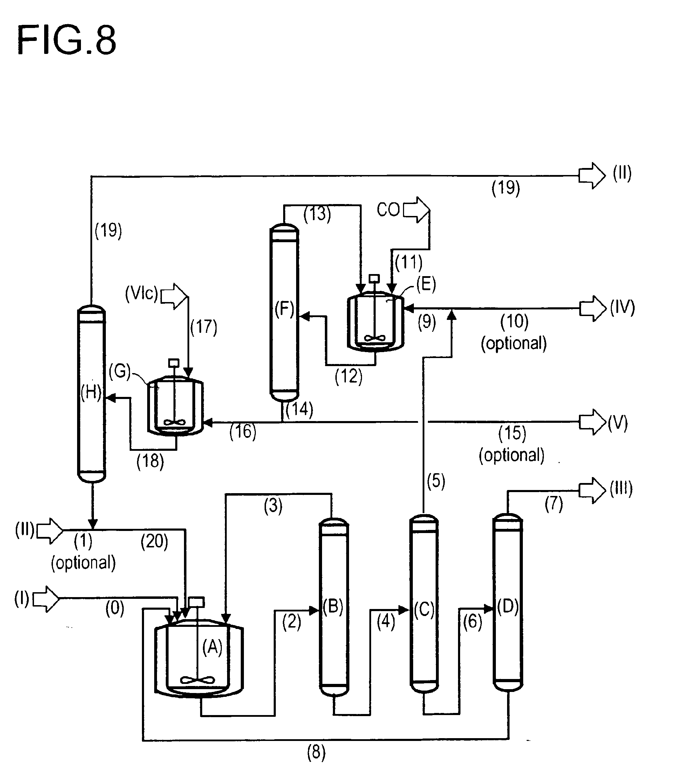

Ein vereinfachtes Verfahrensfließbild ist in Abbildung 8 dargestellt. Der Teil des vereinfachten Verfahrensfließbildes zur Umesterung und zur Carbonylierung entspricht dem in der Ausführungsform 4 dargestelltem Teil. Das gebildetete Essigsäureanhydrid (V) wird über Leitung (16) dem Reaktor (G) zur Acylierung (Alkoholyse) zugeführt. Als Reaktor (G) können alle geeigneten Reaktionsapparate, wie beispielsweise weiter oben unter Schritt (c), Einsatz als Acylierungsreagens (iii)-(aa) beschrieben, eingesetzt werden. Im Reaktor (G) findet in Gegenwart des eingesetzten Katalysators die Alkoholyse zu Essigsäure (II) und dem Essigsäure-C₁- bis C₄-alkylester (VIIc) statt. Über Leitung (17) wird der C₁- bis C₄-Alkanol (VIc) kontinuierlich zugeführt. Das Reaktionsprodukt, welches Essigsäure (II), den Essigsäure-C₁- bis C₄-alkylester (VIIc), gegebenenfalls nicht-umgesetztes Essigsäureanhydrid sowie den eingesetzten Katalysator enthält, wird im Allgemeinen vom Katalysator befreit, beispielsweise in einem Flashverdampfer (der Übersichtlichkeit halber nicht dargestellt) und über Leitung (18) der Kolonne (H) zur destillativen Aufarbeitung zugeführt. Je nach Lage der Siedepunkte, wird der Essigsäure-C₁- bis C₄-alkylester (VIIc) über Kopf (beispielsweise Essigsäure-methyl-, -ethyl- und -propyl-ester) oder über Sumpf (beispielsweise Essigsäurebutylester) von der Essigsäure (II) abgetrennt. Der Essigsäure-C₁- bis C₄-alkylester (VIIc) haltige Strom wird über Leitung (19) abgeführt. Die abgetrennte Essigsäure (II) wird über Leitung (20) wieder dem Reaktor (A) zugeführt, womit sich der Kreislauf schließt.

Ausführungsform 7: Herstellung von Ameisensäure und Essigsäurevinylester (mit Essigsäurekreislauf)

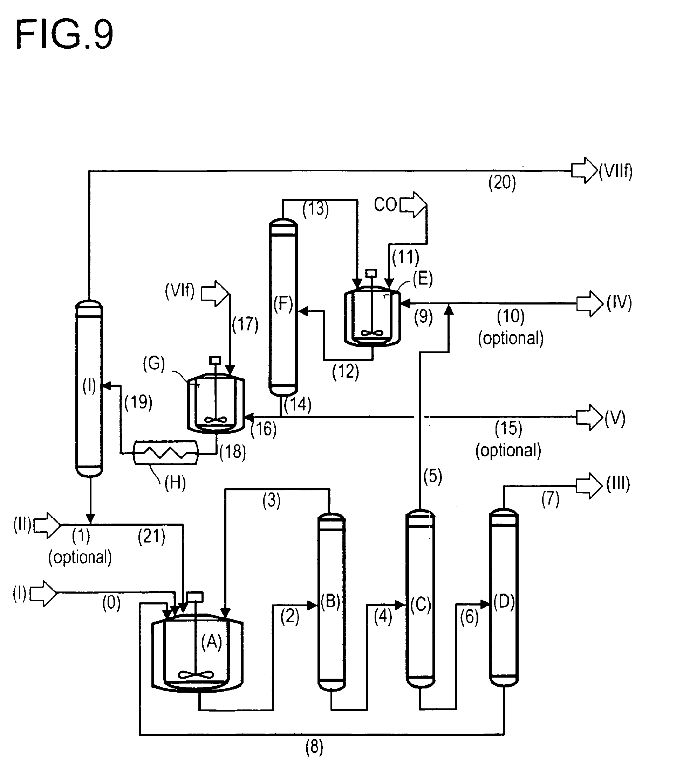

Ein vereinfachtes Verfahrensfließbild ist in Abbildung 9 dargestellt. Der Teil des vereinfachten Verfahrensfließbildes zur Umesterung und zur Carbonylierung entspricht dem in der Ausführungsform 4 dargestelltem Teil. Das gebildetete Essigsäureanhydrid (V) wird über Leitung (16) dem Reaktor (G) zur Acylierung (Addition an Acetaldehyd) zugeführt. Als Reaktor (G) können alle geeigneten Reaktionsapparate, wie beispielsweise weiter oben unter Schritt (c), Einsatz als Acylierungsreagens (iii)-(dd) beschrieben, eingesetzt werden. Im Reaktor (G) findet in Gegenwart des eingesetzten Katalysators die Additionsreaktion zu 1,2-Diacetoxyethan (Ethylenglykoldiacetat) statt. Über Leitung (17) wird hierzu Acetaldehyd (VIf) kontinuierlich zugeführt. Das Reaktionsprodukt wird über Leitung (18) kontinuierlich entnommen und dem Reaktor (H) zur thermischen Zersetzung zugeführt. Als Reaktor (H) können alle geeigneten Reaktionsapparate, wie beispielsweise weiter oben unter Schritt (c), Einsatz als Acylierungsreagens (iii)-(dd) beschrieben, eingesetzt werden. Das entstehende Reaktionsgemisch wird kondensiert und/oder ausgewaschen und in einer weiteren Kolonne (I) in Essigsäurevinylester (VIIf) und Essigsäure (II) getrennt. Der Essigsäurevinylester (VIIf) wird über Kopf entnommen und über Leitung (20) als Produkt abgeführt. Die über Sumpf abgterennte Essigsäure (II) wird wieder dem Reaktor zur Umesterung zugeführt, womit sich der Kreislauf schließt.

Ausführungsform 8: Herstellung von Ameisensäure, Essigsäurevinylester und Essigsäure (mit Essigsäurekreislauf)

Der Verfahrensteil zur Umesterung, zur Carbonylierung und zur Acylierung (Alkoholyse mit 1,2-Ethandiol) entspricht im Wesentlichen dem in der Ausführungsform 6 dargestelltem Teil. Als Alkohol (VIc) wird 1,2-Ethandiol (Ethylenglykol) eingesetzt. Das aus der Alkoholyse stammende Reaktionsgemisch enthält im Wesentlichen 1,2-Diacetoxyethan und Essigsäure (II). Dieses wird im Allgemeinen destillativ von der Essigsäure (II) befreit und das 1,2-Diacetoxyethan einer Thermolysezone zugeführt. Das entstehende Reaktionsgemisch wird kondensiert und/oder ausgewaschen und in einer weiteren Kolonne in Essigsäurevinylester (VIIf) und Essigsäure (II) getrennt. Der Essigsäurevinylester (VIIf) und ein Teil der Essigsäure (II) werden als Produkt entnommen. Der andere Teil der Essigsäure (II) wird wieder dem Reaktor zur Umesterung zugeführt, womit sich der Kreislauf schließt.

Ausführungsform 9: Herstellung von Ameisensäure und Essigsäurevinylester (mit Essigsäurekreislauf)

Der Verfahrensteil zur Umesterung und zur Carbonylierung entspricht im Wesentlichen dem in der Ausführungsform 6 dargestelltem Teil. Das gebildetete Essigsäureanhydrid (V) wird dem Hydrier-/Thermolyse-Reaktor zur reduktiven Umsetzung zugeführt. Als Reaktor können alle geeigneten Reaktionsapparate, wie beispielsweise weiter oben unter Schritt (c), Hydrierung (iv) beschrieben, eingesetzt werden. Im Reaktor findet in Gegenwart des eingesetzten Katalysators und unter Zuführung von Wasserstoff (VIh) und gegebenenfalls von Kohlenmonoxid die Umsetzung zum Essigsäurevinylester und zur Essigsäure (II) statt. Das gebildete Reaktionsgemisch wird im Allgemeinen in einer nachgeschalteten Kolonne in Essigsäurevinylester und Essigsäure (II) getrennt. Der Essigsäurevinylester wird als Produkt entnommen. Die Essigsäure (II) wird wieder dem Reaktor zur Umesterung zugeführt, womit sich der Kreislauf schließt.

Ausführungsform 10: Herstellung von Ameisensäure und Essigsäure (mit Essigsäurekreislauf und vereinfachter Trennung von Ameisensäuremethylester und Essigsäure)

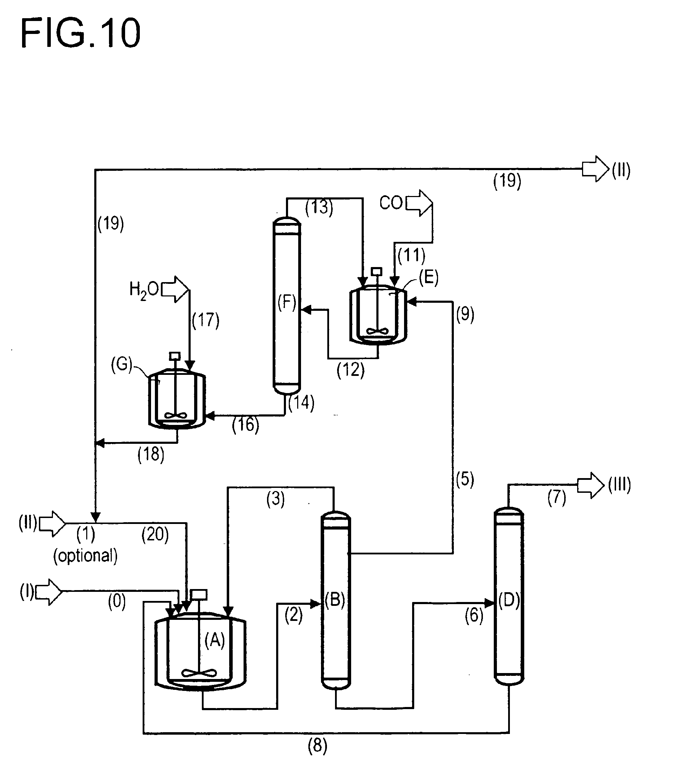

Ein vereinfachtes Verfahrensfließbild ist in Abbildung 10 dargestellt. Ameisensäuremethylester (I) und Essigsäure (II) werden dem Reaktor (A) über Leitung (0) und (20) kontinuierlich zugeführt. Das Reaktionsgemisch, welches Ameisensäuremethylester (I), Essigsäure (II), Ameisensäure (III), Essigsäuremethylester (IV) sowie den eingesetzten Katalysator enthält, wird kontinuierlich dem Reaktor (A) entnommen und über Leitung (2) der vereinfachten destillativen Aufarbeitung, welche in Form der Kolonnen (B) und (D) dargestellt ist, zugeführt. In Kolonne (B) werden die Ester (I) und (IV) von den Säuren (II) und (III) getrennt, wobei der die beiden Säuren enthaltende Strom aus dem Kolonnensumpf entnommen und über Leitung (6) der Kolonne (D) zugeführt wird. In Kolonne (D) wird die Ameisensäure (III) über Kopf abgezogen und über Leitung (7) entnommen. Über Leitung (8) werden nicht-umgesetzte Essigsäure (II), Katalysator und eventuell gebildete Hochsieder dem Reaktor (A) rückgeführt. Ameisensäuremethylester (I) und eventuell gebildete Leichtsieder werden über den Kopf der Kolonne (B) entzogen und über Leitung (3) dem Reaktor rückgeführt. Ein Essigsäuremethylester (IV) und Ameisensäuremethylester (I) enthaltender Strom wird dabei als Seitenabzug im Auftriebsteil der Kolonne (B) entnommen und über Leitung (5) weitergeleitet. Dieser enthält im Allgemeinen bis zu 300 Mol-% Ameisensäuremethylester (I), bezogen auf Essigsäuremethylester (IV).

Der Essigsäuremethylester (IV) und Ameisensäuremethylester (I) enthaltende Strom wird über Leitung (9) dem Reaktor (E) kontinuierlich zugeführt. In Reaktor (E) erfolgt in Gegenwart des eingesetzten Katalysators die Carbonylierung des Essigsäuremethylesters (IV) zum Essigsäureanhydrid (V) sowie die Isomerisierung des Ameisensäuremethylesters (I) zur Essigsäure. Es sei betont, dass dem Reaktor (E) zusätzlich noch durch einen separaten Strom Ameisensäuremethylester (I) zugeführt werden kann. Das Reaktionsgemisch von Reaktor (E), welches nicht-umgesetzten Essigsäuremethylester (IV), nicht-umgesetzten Ameisensäuremethylester (I), das gebildete Essigsäureanhydrid (V) sowie die gebildete Essigsäure und den eingesetzten Katalysator enthält, wird kontinuierlich dem Reaktor (E) entnommen, im Allgemeinen vom Katalysator befreit, beispielsweise in einem Flashverdampfer (der Übersichtlichkeit halber nicht dargestellt) und über Leitung (12) der destillativen Aufarbeitung, welche in Form der Kolonne (F) exemplarisch dargestellt ist, zugeführt. Das Sumpfprodukt der Kolonne (F), welches Essigsäureanhydrid (V), Essigsäure sowie eventuell gebildete Hochsieder enthält, wird über Leitung (14) entnommen und über Leitung (16) dem Reaktor (G), welcher exemplarisch als Rührkessel dargestellt ist, zur Hydrolyse zugeführt. Die Leichtsieder werden über Leitung (13) in den Reaktor zurückgeführt. Die aus dem Hydrolysereaktor (G) stammende Essigsäure (II) wird über Leitung (19) als Produkt kontinuierlich abgeführt sowie die für den Kreislauf erforderliche Menge an Essigsäure (II) über Leitung (20) wieder dem Reaktor (A) zugeführt, womit sich der Kreislauf schließt.

Alternativ kann das Sumpfprodukt der Kolonne (F), welches welches Essigsäureanhydrid (V), Essigsäure (II) sowie eventuell gebildete Hochsieder enthält, in einer weiteren Kolonne in einen Essigsäure (II) enthaltenden Strom und einen das Essigsäureanhydrid (V) sowie die eventuell gebildeten Hochsieder enthaltenden Strom getrennt werden, wobei letzterer dem Hydrolysereaktor (G) zugeführt wird. Die abgetrennte Essigsäure (II) kann dann dem Reaktor (A) zur Umesterung zugeführt werden.

Ausführungsform 11: Herstellung von Ameisensäure und Essigsäureanhydrid (mit Essigsäurekreislauf und vereinfachter Trennung von Ameisensäuremethylester und Essigsäure).

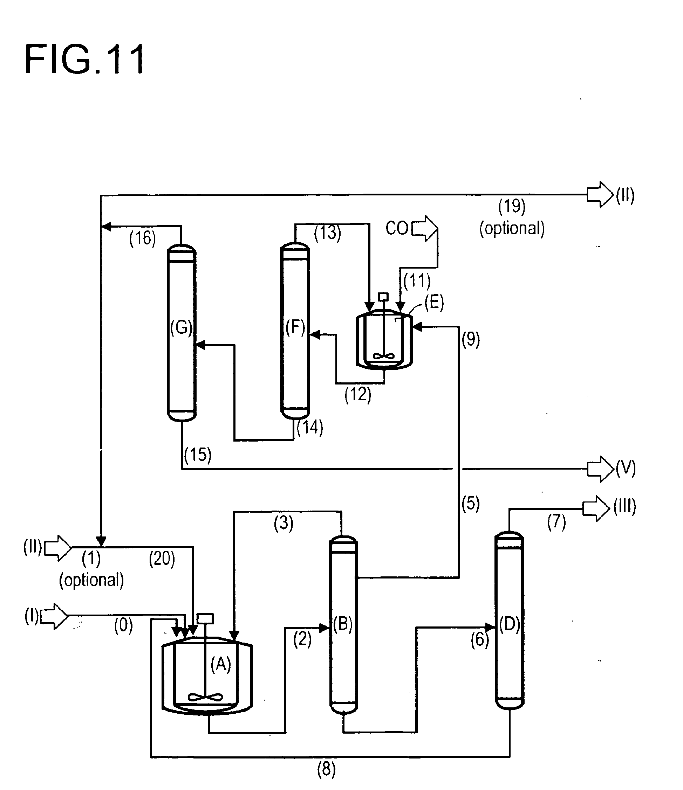

Ein vereinfachtes Verfahrensfließbild ist in Abbildung 11 dargestellt. Die Umesterung sowie die Aufarbeitung des Reaktionsgemisches wird wie in Ausführungsform 10 beschrieben durchgeführt, worauf explizit verwiesen sei.

Der Essigsäuremethylester (IV) und Ameisensäuremethylester (I) enthaltende Strom wird über Leitung (9) dem Reaktor (E) kontinuierlich zugeführt. Dieser Strom.enthält im Allgemeinen bis zu 300 Mol-% Ameisensäuremethylester (I), bezogen auf Essigsäuremethylester (IV). In einer ganz besonders bevorzugten Ausführungsform enthält dieser Strom diejenige Menge an Ameisensäuremethylester (I), welche zur Erzeugung der für den Essigsäure-Kreislauf erforderlichen Menge an Essigsäure erforderlich ist. Dies entspricht einem stöchiometrischen Gehalt von 100 Mol-%, Ameisensäuremethylester (I), bezogen auf Essigsäuremethylester (IV), wobei der einzustellende Wert entsprechend möglicher Verluste auch darüber oder darunter liegen kann. Somit wird die für den Kreislauf erforderliche Essigsäure (II) durch Umesterung von Ameisensäuremethylester (I) erzeugt.

Die Carbonylierung beziehungsweise Isomerisierung erfolgt wie in Ausführungsform 10 beschrieben, worauf explizit verwiesen sei. Im Unterschied zur Ausführungsform 10 wird jedoch das Sumpfprodukt der Kolonne (F), welches Essigsäureanhydrid (V), Essigsäure (II) sowie eventuell gebildete Hochsieder enthält, über Leitung (14) der Kolonne (G) zugeführt, in der eine Trennung in einen Essigsäure (II) enthaltenden und einen Essigsäureanhydrid (V) enthaltenden Strom erfolgt. Über Leitung (15) wird dann das Essigsäureanhydrid (V) als Produkt abgeführt. Der Essigsäure (II) enthaltende Strom wird über Leitung (16) und (20) zum Umesterungsreaktor (A) zurückgeführt. Je nach der Menge an gebildeter Essigsäure ist optional auch eine Entnahme über Leitung (19) möglich.

Alternativ ist es bei dieser Ausführungsform beispielsweise auch möglich, zusätzlich Methanol zur weiteren Erhöhung des Anteils an Essigsäure in den Carbonylierungsreaktor zuzufahren. In einer besonders bevorzugten Ausführungsform wird die für den Kreislauf erforderliche Essigsäure durch die Isomerisierung des Essigsäuremethylesters sowie gegebenenfalls durch die Carbonylierung von zusätzlich zugegebenen Methanols gewonnen.

Das erfindungsgemäße Verfahren ermöglicht die Herstellung von Ameisensäure sowie einer Carbonsäure mit mindestens zwei Kohlenstoffatomen und/oder deren Derivaten, auf Basis einer gut zugänglichen und wirtschaftlich attraktiven Rohstoffbasis. So basieren beispielsweise die besonders bevorzugten Produkte Ameisensäure, Essigsäuremethylester, Essigsäureanhydrid, Essigsäure, Keten vollständig auf Synthesegas und somit auf Erdgas als Rohstoff. Beim besonders bevorzugten Essigsäurevinylester ist beispielsweise bei der Variante zur Hydrierung des Essigsäureanhydrids ebenfalls eine vollständige Erdgasbasis möglich. Je nach Herkunft des Ethanols ist auch bei der Herstellung des besonders bevorzugten Essigsäureethylesters eine vollständige Erdgasbasis möglich.

Weiterhin ermöglicht das erfindungsgemäße Verfahren eine einfache und kostengünstige Gestaltung der Anlage (niedrige Investitionskosten), einen niedrigen Energieverbrauch und niedrige Betriebskosten. Durch die Kopplung der Herstellung von Ameisensäure und einer Carbonsäure mit mindestens zwei Kohlenstoffatomen und/oder deren Derivaten weist eine nach dem erfindungsgemäßen Verfahren arbeitende Anlage einen deutlich niedrigeren Kapitaleinsatz auf als zwei getrennte Anlagen nach dem Stand der Technik. Insbesondere entfällt bei der Herstellung von Essigsäureanhydrid nach erfindungsgemäßen Verfahren der Umweg über giftiges und in der Herstellung energieintensives Keten.

Das erfindungsgemäße Verfahren vermeidet die Bildung unerwünschter Nebenprodukte infolge Koppelproduktion.

Des weiteren ermöglicht das erfindungsgemäße Verfahren bei Bedarf auch die Herstellung wasserfreier Ameisensäure und wasserfreier Carbonsäuren, welche eine deutlich geringere Korrosionsaggresivität aufweisen als die wasserhaltigen Verbindungen, somit eine höhere Sicherheit bieten und den Einsatz kostengünstigerer Konstruktionsmaterialen ermöglichen. Durch den gegenüber dem Stand der Technik einfachen und wirtschaftlich attraktiven Zugang zu wasserfreier Ameisensäure wird eine besonders hohe Ameisensäure-Qualität erreicht. Durch die damit verbundene Erhöhung der Ameisensäurekonzentration auf bis zu 100% resultiertiert hierbei auch ein Vorteil beim Transport und der Lagerung.

Ferner bietet das erfindungsgemäße Verfahren ein hohes Maß an Flexibilität auf der Seite der Carbonsäure mit mindestens zwei Kohlenstoffatomen und/oder deren Derivaten, da die relativen Mengen der ausgeschleusten Verbindungen je nach Bedarf in einem breiten Bereich variieren können. Durch eine zusätzliche Zufuhr eines Alkohols zur Carbonylierungsstufe kann das Verhältnis der Carbonylierungsprodukte zur Ameisensäure erhöht werden. Somit besteht auch in Bezug auf eine Mehrproduktion an Carbonylierungsprodukten und dessen Folgeprodukten ein hohes Maß an Flexibilität.

Bei der bevorzugten Darstellung von Essigsäure und deren Derivaten bietet das erfindungsgemäße den weiteren Vorteil, die Carbonylierung des Essigsäuremethylesters in Abwesenheit von Wasser durchzuführen und somit gegenüber der technisch üblichen Carbonylierung von Methanol durch Vermeidung der Wassergas-Shift-Reaktion eine höhere Ausbeute des eingesetzten Kohlenmonoxids zu erreichen.

**Tabelle 1:**

| Bevorzugte Ausführungsformen unter Berücksichtigung der idealisierten stöchiometrischen Verhältnisse. | | | |
|---|---|---|---|
| | Edukte | Produkte | Verfahrensblöcke |
| 1 | (I):Ameisensäuremethylester | (III): Ameisensäure | A, C |
| | (II): Essigsäure | (V): Essigsäureanhydrid | |
| | Kohlenmonoxid | | |
| 2 | (I): Ameisensäuremethylester | (III): Ameisensäure | A, B, C, E (Hydrolyse) |
| | Kohlenmonoxid | (IV): Essigsäuremethylester | Essigsäure (II) im Kreislauf |
| | (VI): Wasser | | |
| 3 | (I): Ameisensäuremethylester | (III): Ameisensäure | A, C, D, E (Hydrolyse) |
| | Kohlenmonoxid | (V): ½ Essigsäureanhydrid | Essigsäure (II) im Kreislauf |
| | (Vl): ½ Wasser | | |
| 4 | (I): Ameisensäuremethylester | (III): Ameisensäure | A, C, E (Hydrolyse), F |
| | Kohlenmonoxid | (VI): Essigsäure | Essigsäure (II) im Kreislauf |
| | (VI): Wasser | | |
| 5 | (I): Ameisensäuremethylester | (III): Ameisensäure | A, C, E (thermische Zersetzung) |
| | Kohlenmonoxid | (VI): Keten | Essigsäure (II) im Kreislauf |
| 6 | (I): Ameisensäuremethylester | (III): Ameisensäure | A, C, E (Acylierung) |
| | Kohlenmonoxid | (VI): Essigsäure-C₁- bis C₄-alkylester | Essigsäure (II) im Kreislauf |
| | (VI): C₁- bis C₄-Alkanol | | |
| 7 | (I): Ameisensäuremethylester | (III): Ameisensäure | A, C, E (Acylierung) |
| | Kohlenmonoxid | (VI): Essigsäurevinylester | Essigsäure (II) im Kreislauf |
| | (VI): Acetaldehyd | | |
| 8 | (I): Ameisensäuremethylester | (III): Ameisensäure | A, C, E (Acylierung) |
| | Kohlenmonoxid | (Vl): ½ Essigsäurevinylester + ½ Essigsäure | Essigsäure (II) im Kreislauf |
| | (VI): ½ 1,2-Ethandiol | | |
| 9 | (I): Ameisensäuremethylester | (III): Ameisensäure | A, C, E (Hydrierung) |
| | Kohlenmonoxid | (VI): ½ Essigsäurevinylester | Essigsäure (II) im Kreislauf |
| | (V1): ½ Wasserstoff | | |
| 10 | (I): Ameisensäuremethylester | (III): Ameisensäure | A, C, E (Hydrolyse), F |
| | Kohlenmonoxid | (VI): n Essig | Essigsäure (II) im Kreislauf |
| | (VI): Wasser | | (n > 1) |
| 11 | (I): Ameisensäuremethylester | (III): Ameisensäure | A, C, D, E (Hydrolyse) |
| | Kohlenmonoxid | (VI): n/2 Essigsäureanhydrid | Essigsäure (II) im Kreislauf |
| | (VI): (2-n)/2 Wasser | | (1 > n ≥ 2) |

## Patentansprüche

1. Verfahren zur gemeinsamen Herstellung von Ameisensäure sowie einer Carbonsäure mit mindestens zwei Kohlenstoffatomen und/ oder deren Derivaten, **dadurch gekennzeichnet, dass** man
(a) einen Ameisensäureester (I) mit einer Carbonsäure mit mindestens zwei Kohlenstoffatomen (II) zu Ameisensäure (III) und dem entsprechenden Carbonsäureester (IV) umestert; und
(b) mindestens einen Teil des in Schritt (a) gebildeten Carbonsäureesters (IV) zum entsprechenden Carbonsäureanhydrid (V) carbonyliert.

2. Verfahren nach Anspruch 1, **dadurch gekennzeichnet, dass** man
(c) mindestens einen Teil des in Schritt (b) gebildeten Carbonsäureanhydrids (V) in die Carbonsäure (II) überführt.

3. Verfahren nach Anspruch 2, **dadurch gekennzeichnet, dass** man das Carbonsäureanhydrid (V) durch thermische Zersetzung, durch Hydrolyse, durch den Einsatz als Acylierungsreagens und/oder durch Hydrierung in die Carbonsäure (II) überführt.

4. Verfahren nach Anspruch 3, **dadurch gekennzeichnet, dass** man die Carbonylierung von Schritt (b) und die Hydrolyse von Schritt (c) zusammen in einem Reaktionsapparat durchführt.

5. Verfahren nach Anspruch 3, **dadurch gekennzeichnet, dass** man das Carbonsäureanhydrid (V) gegenüber einem Aldehyd oder Keton als Acylierungsreagens unter Bildung eines α,β-ungesättigten Carbonsäureesters einsetzt.

6. Verfahren nach Anspruch 3, **dadurch gekennzeichnet, dass** man das Carbonsäureanhydrid (V) gegenüber einem Alkohol als Acylierungsreagens unter Bildung eines Carbonsäureesters einsetzt.

7. Verfahren nach Anspruch 3, **dadurch gekennzeichnet, dass** man die Carbonylierung von Schritt (b) und die Hydrierung von Schritt (c) zusammen in einem Reaktionsapparat durchführt.

8. Verfahren nach den Ansprüchen 2 bis 7, **dadurch gekennzeichnet, dass** man mindestens einen Teil der in Schritt (c) gebildeten Carbonsäure (II) in Schritt (a) einsetzt.

9. Verfahren nach den Ansprüchen 1 bis 8, **dadurch gekennzeichnet, dass** man als Ameisensäureester (I) Ameisensäuremethylester einsetzt.

10. Verfahren nach den Ansprüchen 1 bis 9, **dadurch gekennzeichnet, dass** man als Carbonsäure (II) Essigsäure einsetzt.

11. Verfahren nach den Ansprüchen 9 bis 10, **dadurch gekennzeichnet, dass** man Ameisensäure sowie Essigsäuremethylester, Essigsäureanhydrid, Essigsäure, Keten, Essigsäurevinylester, Essigsäureethylester, Essigsäurepropylester und/oder Essigsäurebutylester herstellt.

## Claims

1. A process for the joint preparation of formic acid and a carboxylic acid having at least two carbon atoms and/or derivatives thereof, wherein
(a) a formic ester (I) is transesterified with a carboxylic acid having at least two carbon atoms (II) to form formic acid (III) and the corresponding carboxylic ester (IV); and
(b) at least part of the carboxylic ester (IV) formed in step (a) is carbonylated to give the corresponding carboxylic anhydride (V).

2. The process according to claim 1, wherein
(c) at least part of the carboxylic anhydride (V) formed in step (b) is converted into the carboxylic acid (II).

3. The process according to claim 2, wherein the carboxylic anhydride (V) is converted into the carboxylic acid (II) by thermal decomposition, by hydrolysis, by use as acylating reagent and/or by hydrogenation.

4. The process according to claim 3, wherein the carbonylation of step (b) and the hydrolysis of step (c) are carried out together in one reaction apparatus.

5. The process according to claim 3, wherein the carboxylic anhydride (V) is used as acylating reagent in a reaction with an aldehyde or ketone to form an α,β-unsaturated carboxylic ester.

6. The process according to claim 3, wherein the carboxylic anhydride (V) is used as acylating reagent in a reaction with an alcohol to form a carboxylic ester.

7. The process according to claim 3, wherein the carbonylation of step (b) and the hydrogenation of step (c) are carried out together in one reaction apparatus.

8. The process according to any of claims 2 to 7, wherein at least part of the carboxylic acid (II) formed in step (c) is used as starting material in step (a).

9. The process according to any of claims 1 to 8, wherein the formic ester (I) used is methyl formate.

10. The process according to any of claims 1 to 9, wherein the carboxylic acid (II) used is acetic acid.

11. The process according to any of claims 9 to 10, wherein formic acid is prepared together with methyl acetate, acetic anhydride, acetic acid, ketene, vinyl acetate, ethyl acetate, propyl acetate and/or butyl acetate.

## Revendications

1. Procédé de production simultanée d'acide formique et d'un acide carboxylique avec au moins deux atomes de carbone et/ou leurs dérivés, **caractérisé:**
(a) **en ce qu'**un ester d'acide formique (I) est estérifié avec un acide carboxylique avec au moins deux atomes de carbone (II) en acide formique (III) et en ester d'acide carboxylique correspondant (IV) et
(b) qu'au moins une partie de l'ester d'acide carboxylique (IV) formé dans l'étape (a) est carbonylée en anhydride d'acide carboxylique correspondant (V).

2. Procédé selon la revendication 1, **caractérisé :**
(c) **en ce qu'**au moins une partie de l'anhydride d'acide carboxylique (V) formé dans l'étape (b) est transformée en acide carboxylique (II).

3. Procédé selon la revendication 2, **caractérisé en ce que** l'anhydride d'acide carboxylique (V) est transformé en acide carboxylique (II) par décomposition thermique, par hydrolyse, par l'utilisation comme réactif d'acylation et/ou par hydrogénation.

4. Procédé selon la revendication 3, **caractérisé en ce que** la carbonylation de l'étape (b) et l'hydrolyse de l'étape (c) sont effectuées simultanément dans un appareil de réaction.

5. Procédé selon la revendication 3, **caractérisé en ce que** l'anhydride d'acide carboxylique (V) est utilisé par rapport à un aldéhyde ou une cétone comme réactif d'acylation en formant un ester d'acide carboxylique α,β-insaturé.

6. Procédé selon la revendication 3, **caractérisé en ce que** l'anhydride d'acide carboxylique (V) est utilisé par rapport à un alcool comme réactif d'acylation en formant un ester d'acide carboxylique.

7. Procédé selon la revendication 3, **caractérisé en ce que** la carbonylation de l'étape (b) et l'hydrogénation de l'étape (c) sont effectuées simultanément dans un appareil de réaction.

8. Procédé selon les revendications 2 à 7, **caractérisé en ce qu'**au moins une partie de l'acide carboxylique (II) formé dans l'étape (c) est utilisée dans l'étape (a).

9. Procédé selon les revendications 1 à 8, **caractérisé en ce que** le méthylester d'acide formique est utilisé comme ester d'acide formique (I).

10. Procédé selon les revendications 1 à 9, **caractérisé en ce que** l'acide acétique est utilisé comme acide carboxylique (II).

11. Procédé selon les revendications 9 à 10, **caractérisé en ce que** de l'acide formique ainsi que du méthylester acétique, de l'anhydride acétique, de l'acide acétique, du cétène, du vinylester acétique, de l'éthylester acétique, du propylester acétique et/ou du butylester acétique sont synthétisés.
